# EUROPEAN PATENT APPLICATION

(11) **EP 3 499 230 A1**
(43) Date of publication of application: **19.06.2019**
(21) Application number: 17839323.7
(22) Date of filing: 03.08.2017
(51) Int. Cl.: G01N 33/493, G01N 33/53, G01N 33/68

(54) **METHOD FOR ASSISTING DIAGNOSIS OF ALZHEIMER'S DISEASE USING URINE BIOMARKER**

(30) Priority: 09.08.2016 JP 2016156820
(71) Applicant: Otsuka Pharmaceutical Co., Ltd., Tokyo 101-8535 (JP)
(72) Inventor: KATSURAGI, Kiyonori, Osaka-shi Osaka 540-0021 (JP); MACHIDA, Kiyotaka, Osaka-shi Osaka 540-0021 (JP); KARIYAZONO, Hirokazu, Osaka-shi Osaka 540-0021 (JP); HIGASHIYAMA, Ryo, Osaka-shi Osaka 540-0021 (JP); KOBAYASHI, Hironori, Osaka-shi Osaka 540-0021 (JP); SAIJO, Yoko, Osaka-shi Osaka 540-0021 (JP); SATO, Ayumi, Osaka-shi Osaka 540-0021 (JP)
(74) Representative: dompatent von Kreisler Selting Werner - Partnerschaft von Patent- und Rechtsanwälten mbB
(86) International application number: PCT/JP2017/028173
(87) International publication number: WO 2018/030252

(57) **Abstract**

An object of the invention is to provide a method for assisting diagnosis of Alzheimer's disease (AD), and a detection reagent, a diagnosis kit and a diagnosis system for use in the method. Provided is a method for assisting diagnosis of AD, comprising the steps of: measuring an amount of a urine biomarker in a urine sample derived from urine collected from a subject; and determining whether the subject suffers from AD or has a high risk of developing AD based on the amount of the urine biomarker measured, wherein the urine biomarker is at least one urine protein selected from the group consisting of ApoA-I, ApoA-II, ApoA-IV, ApoB-100, ApoB-48, ApoC-I, ApoC-II, ApoC-III, ApoD, ApoE, IFITM1, IFITM2, IFITM3, NPC1, NPC2, NPC1L1, and MT.

## Description

### Technical Field

The present invention relates to a method for assisting diagnosis of Alzheimer's disease, and a detection reagent, a diagnosis kit and a diagnosis system for use in the method.

### Background Art

Alzheimer's disease (AD) is a progressive neurodegenerative disease to cause memory impairment and dementia, and is said to account for the largest fraction of patients with dementia. AD destroys the personality of a patient, not only causing memory impairment, to result in loss of the social life function of the patient, and hence is a disease to impose a heavy burden not only to an AD patient but also to his or her family members. In Japan, where population aging is progressing, the number of patients with AD and other dementias is rapidly increasing, which is recognized as a serious social problem.

Attention has been focused on mild cognitive impairment (MCI) as a pre-stage of dementia in recent years. MCI is caused by various factors, and amnestic MCI, a subtype of MCI, is reported to progress to AD with a high probability. In the current situation that no fundamental therapy for AD has been found, the importance of carrying out therapeutic intervention once any AD lesion has been found, even without any symptom, has been discussed to prevent dementia caused by AD. One of the backgrounds of such discussion is the success of biomarker studies in estimating brain pathology of AD.

Examples of diagnosis methods widely used for diagnosis of dementias including AD include Hasegawa Dementia Scale-Revised (HDS-R) and Mini-Mental State Examination (MMSE), each based on interview of individuals to be examined. These interviewing methods are used for the purpose of screening for dementia. Imaging examination is further used to discriminate AD from other dementias. The U.S. National Institute on Aging/Alzheimer's Association (NIA/AA) presented new diagnostic criteria for AD in 2011. The NIA/AA employs diagnostic criteria based on biomarkers in addition to clinical diagnostic criteria based on clinical findings.

Examples of AD diagnosis based on biomarkers include imaging examination based on neuroimaging biomarkers (e.g., quantification of atrophy of the medial temporal lobe through functional magnetic resonance imaging (fMRI)). In imaging examination performed in AD diagnosis, imaging apparatuses for MRI, computed tomography (CT), positron emission tomography (PET), or the like are commonly used. Since these imaging apparatuses require special equipment, only certain facilities can implement such imaging examination. Hence, individuals to be tested need to visit a particular medical institution for imaging examination. In addition, it takes a long time (approximately 2 hours) for the examination. Thus, imaging examination for AD diagnosis is an examination geographically and temporally constrained, and far from simple. Moreover, it costs several tens of thousands of yen per examination (Non-Patent Literature 1).

Examples of biochemical biomarkers for diagnosis of AD include decrease of the amyloid-beta (Aβ) 42 level or increase of the phosphorylated tau protein level in cerebrospinal fluid (CSF). An ELISA kit for measurement of phosphorylated tau protein in CSF (Non-Patent Literature 2) has been placed on the market, and increase of the phosphorylated tau protein level in CSF has been practically used as a biomarker. However, this method requires use of a puncture needle in collecting CSF to impose a heavy physical burden to an individual to be tested (high invasiveness), and hence is not suitable for continuously monitoring the condition of an individual to be tested through repeated examinations.

Blood is a biological sample such that invasiveness in collecting is lower than that in collecting CSF. It has been reported that blood concentrations of specific apolipoprotein were statistically significantly different between an AD group and a non-AD group (Non-Patent Literatures 3 to 6). However, the difference is insufficient for practical use as a biomarker, and in collecting blood a heavy physical burden is imposed to an individual to be examined, similarly.

Extracellular vesicles (EVs) have been receiving attention in recent years. An extracellular vesicle is a nano-sized to micro-sized vesicle which is surrounded by a lipid bilayer membrane and discharged from a cell. Extracellular vesicles have been found to retain in the inside not only intracellular proteins but also micro RNA, which exhibits an important function for suppression of gene expression in the living body, and hence have attracted attention as an intercellular communication tool. Extracellular vesicles are also present in body fluid such as blood, urine, and breast milk, thus attracting attention as a biological sample for search for biomarkers (Non-Patent Literature 7).

### Citation List

### Non-Patent Literature

Non-Patent Literature 1: Guideline for treatment of dementia 2010, Igaku-Shoin Ltd. (supervisor: Japanese Society of Neurology)
Non-Patent Literature 2: ARAI, Hiroyuki et al., The Journal of Clinical Laboratory Instruments and Reagents (2013) 36(5): 713-717
Non-Patent Literature 3: Caramelli et al., Acta Neurol Scand (1999) 100: 61-63
Non-Patent Literature 4: Taddei et al., Neuroscience Letters (1997) 223: 29-32
Non-Patent Literature 5: Cudaback et al., Journal of Neuroinflammation (2012) 9(192): 1-13
Non-Patent Literature 6: Uchida et al., Diagnosis, Assessment & Disease Monitoring (2015) 1: 270-280
Non-Patent Literature 7: SHIMODA, Asako et al., Drug Delivery System (2014) 29-2: 108-115

### Summary of Invention

### Technical Problem

In the field, there is requirement of a method for assisting diagnosis of AD which imposes almost no physical burden to individuals to be tested and can be implemented in a simple manner.

Many kinds of proteins which possibly serve as biomarkers are present in blood at high concentrations. Accordingly, blood is typically used for search for disease biomarkers. The amounts of proteins contained in urine (hereinafter, referred to as "urine proteins") are trivial as compared with the amounts of proteins in blood. Most of the urine proteins are believed to be derived from organs relating to urination such as the kidney and the bladder, and, as a matter of fact, there is no report on AD with focus on urine, as far as the present inventors know.

The present inventors diligently examined biomarkers for AD diagnosis, and surprisingly found urine proteins the amounts of which significantly differ between an AD group and a non-AD group, thus completing the present invention. The findings provided herein by the present inventors suggest the possibility that AD-associated molecules in blood are selectively concentrated in the course of excretion thereof from blood through urine; however, the present invention is by no means limited by such hypothesis or possibility.

### Solution to Problem

The present invention provides a method for assisting diagnosis of Alzheimer's disease, and a detection reagent, a diagnosis kit, and a diagnosis system for use in the method, as described in the following.

A first aspect of the present invention provides a method for assisting diagnosis of Alzheimer's disease, the method including the steps of: (i) measuring the amount of a urine biomarker in a urine sample derived from urine collected from a subject; and (ii) determining whether the subject suffers from AD or has a high risk of developing AD based on the amount of the urine biomarker measured, wherein the urine biomarker is at least one urine protein selected from the group consisting of Apolipoprotein (hereinafter, abbreviated as "Apo") A-I, ApoA-II, ApoA-IV, ApoB-100, ApoB-48, ApoC-I, ApoC-II, ApoC-III, ApoD, ApoE, Interferon-induced transmembrane protein (hereinafter, abbreviated as "IFITM") 1, IFITM2, IFITM3, Neimann-Pick C (hereinafter, abbreviated as "NPC") 1, NPC2, NPC1L1, and Metallothionein (hereinafter, abbreviated as "MT").

In the step (i), for example, a urine sample possibly containing a urine biomarker and a reagent for detecting the urine biomarker are mixed together to form a conjugate of the urine biomarker with the reagent. Subsequently, for example, the conjugate is separated from the urine biomarker or reagent left unreacted (B/F separation). In the case that the reagent contains a fluorescent substance or a luminescent substance as a labeling substance, a signal of fluorescence or luminescence is detected from the conjugate. If the conjugate is formed in a manner depending on (e.g., in proportion to) the amount of the urine biomarker in the urine sample, a quantitative parameter (e.g., fluorescence intensity or luminescence intensity) for the signal can reflect the amount of the urine biomarker in the urine sample. From the quantitative parameter for the signal, the amount (e.g., the concentration or content) of the urine biomarker in the urine sample can be calculated.

In the step (ii), determination is made on whether a provider of the urine sample measured (subject) suffers from AD or has a high risk of developing AD based on measured values of the quantitative parameter measured in the step (i) or the concentration or content of the urine biomarker calculated from the measured values. For example, the amount of the urine biomarker measured in the step (i) is compared with a threshold corresponding to the amount of the urine biomarker, and the subject is determined to suffer from AD or have a high risk of developing AD if the amount of the urine biomarker measured is higher than the threshold. The threshold is, for example, a value to discriminate a pre-set AD group and non-AD group.

A second aspect of the present invention provides a detection reagent for use in the method according to the first aspect. The detection reagent contains: at least one probe selected from the group consisting of antibodies, antibody fragments, single-chain antibodies, and aptamers, each for at least one urine biomarker selected from the group consisting of ApoA-I, ApoA-II, ApoA-IV, ApoB-100, ApoB-48, ApoC-I, ApoC-II, ApoC-III, ApoD, ApoE, IFITM1, IFITM2, IFITM3, NPC1, NPC2, NPC1L1, and MT. The detection reagent may further contain at least one labeling substance selected from the group consisting of fluorescent substances, radioactive substances, and enzymes, in addition to the probe.

A third aspect of the present invention provides a diagnosis kit for use in the method according to the first aspect, the kit including the detection reagent according to the second aspect. The diagnosis kit further includes a carrier such as a plate and beads, and/or a reagent required in addition to the detection reagent. The diagnosis kit may further include an accompanying document. The accompanying document describes, for example, how to use the reagents and determination criteria. The determination criteria in the description is, for example, such that a subject is determined to suffer from AD or have a high risk of developing AD if the measured amount of a urine biomarker in a urine sample is higher than a threshold corresponding to the amount of the urine biomarker.

A fourth aspect of the present invention provides a diagnosis system for AD including: a determination section configured to determine whether a subject suffers from AD or has a high risk of developing AD by comparing an amount of a urine biomarker in a urine sample derived from urine collected from the subject with a threshold corresponding to the amount of the urine biomarker with respect to AD; and an indication section configured to indicate a determination result from the determination section, wherein the urine biomarker is at least one urine protein selected from the group consisting of ApoA-I, ApoA-II, ApoA-IV, ApoB-100, ApoB-48, ApoC-I, ApoC-II, ApoC-III, ApoD, ApoE, IFITM1, IFITM2, IFITM3, NPC1, NPC2, NPC1L1, and MT. The system may include a database storing thresholds corresponding to a plurality of the urine biomarkers selected from the above-described group, and the determination section may refer to information including types of the urine biomarker in the urine sample derived from the urine collected from the subject and information on whether the disease to be determined is either AD or heart disease or both AD and heart disease to acquire the corresponding threshold from the database based on the pieces of information, and make determination based on the threshold acquired.

### Advantageous Effects of Invention

The method for assisting diagnosis according to the present invention uses urine as a biological sample, and hence imposes almost no physical burden in collecting (non-invasive).

The method for assisting diagnosis according to the present invention, which uses a urine sample, can be implemented in a non-invasive and simple manner, in contrast to examination based on biomarkers in CSF. Accordingly, the method for assisting diagnosis according to the present invention is suitable for continuously monitoring the condition of a subject through repeated examinations. The method for assisting diagnosis according to the present invention can be implemented in a simple manner without being geographically or temporally constrained, in contrast to imaging examination. The method for assisting diagnosis according to the present invention can be advantageous also in cost because of no need of practice by a physician in collecting a sample and of an expensive imaging apparatus.

### Brief Description of Drawings

Figure 1 shows dot plots to compare concentrations of urine proteins (a) ApoB-100, (b) ApoE, (c) ApoC-I, (d) ApoA-I, (e) IFITM2/3, (f) NPC1, and (g) MT in urine samples derived from urines collected from an Alzheimer's disease (AD) group, a heart disease (coronary heart disease: CHD) group, and a healthy subject (HS) group.
Figure 2 shows bar graphs regarding methods for assisting diagnosis using combination of two urine biomarkers selected from (a) ApoB-100, (b) ApoE, (c) IFITM2/3, and (d) MT.
Figure 3 shows line graphs to show the influence of creatinine correction on the variation of the amount of a urine protein.
Figure 4 shows line graphs to show the influence of enrichment (concentration) treatment in preparing urine samples.
Figure 5 shows line graphs of the amounts of urine proteins (a) ApoB-100, (b) IFITM2/3, and (c) MT collected with extraction treatment (alkaline process or freezing process) or without extraction treatment (untreated) in preparing urine samples, to demonstrate the influence of the extraction treatment on the amounts collected.
Figure 6 shows bar graphs regarding methods for assisting diagnosis using co-localization of two urine biomarkers (top: ApoB-100 and ApoE, bottom: ApoB-100 and ApoC-I) as an index.
Figure 7 shows a block diagram illustrating the schematic configuration of a diagnosis system as an overall view.
Figure 8 shows a flow chart illustrating a diagnosis flow for AD in the diagnosis system.
Figure 9 shows a table of an example of threshold data with respect to AD stored in a database.
Figure 10 shows a flow chart illustrating a diagnosis flow for heart disease in the diagnosis system.
Figure 11 shows a table of an example of threshold data with respect to heart disease stored in a database.

### Description of Embodiments

AD is a progressive neurodegenerative disease to result in dementia. In the present specification, AD is occasionally distinguished into "dementia caused by AD" and "mild cognitive impairment caused by AD" by the pathological condition. Dementia or mild cognitive impairment caused by AD is diagnosed as dementia or mild cognitive impairment according to known clinical diagnosis criteria, and presents as pathological condition diagnosed as AD according to diagnosis criteria based on biomarkers.

Examples of the "subject" include mammals such as dogs, cattle, sheep, non-human primates, and humans. The subject is preferably a human. In an embodiment, the subject is a human diagnosed with AD or human diagnosed with mild cognitive impairment, according to clinical diagnosis criteria. In another embodiment, the subject is a human for which no sign of dementia is found. In another embodiment, the subject is a human diagnosed with dementia caused by AD or human diagnosed with mild cognitive impairment caused by AD.

In the present specification and appended claims, the term "urine sample" refers to a sample to be subjected to measurement. The urine sample may be urine collected from a subject, or a sample subjected to extraction treatment, described later, to extract urine protein from a complex (including extracellular vesicles) containing urine protein. The urine sample may be a sample enriched with urine protein present in the complex and/or urine protein in a free form. The method for collecting urine is not limited. The urine may be pooled urine for one day or spot urine. In using spot urine, the variation of the amount of urine protein in the spot urine may be corrected. Examples of methods for correcting the variation of the amount of urine protein include, but are not limited to, urinary creatinine correction. In an embodiment, the urine sample is spot urine or a sample prepared from spot urine. The urine sample derived from urine collected from a subject may contain an additive, in a manner such that the additive does not interfere with measurement of urine protein. Examples of such additives include, but are not limited to, buffers, protease inhibitors, pH adjusters, surfactants, and chelating agents.

The term "urine protein" refers to protein found in urine collected from a subject, and the term does not discriminate by the state. For example, urine protein may be present in a free form in urine collected from a subject, or be forming a complex with lipid or other protein.

The term "urine protein in a free form" refers to urine protein which can be present only as protein molecules or as holoprotein, which includes a non-protein molecule as a cofactor, in urine or a urine sample. The term "complex containing urine protein" or "urine protein-containing complex" refers to a complex of a urine protein with another component, and examples thereof include, but are not limited to, conjugates of a specific urine protein with other urine protein, complexes of urine protein with phospholipid (lipoprotein), and extracellular vesicles containing urine protein. In the complex, at least one, for example, one, two, or three or more urine proteins may be present, though the number of urine proteins is not limited thereto. In the present specification and appended claims, in the situation that two or more urine proteins are present in one complex, the urine biomarkers are said to be "co-localized" in the complex.

The term "extracellular vesicle" refers to a particle surrounded by a lipid bilayer membrane, and the term does not discriminate by the generation mechanism or size. Extracellular vesicles containing at least one urine protein each include the urine protein in the inside of a lipid bilayer membrane, or on a lipid bilayer membrane, or in the inside of and on a lipid bilayer membrane, in accordance with the type of the urine protein and the condition of a subject, though the mode of inclusion is not limited thereto.

The term "urine biomarker" refers to a urine protein or partial peptide thereof the content or concentration of which can change in relation to AD and which is an apolipoprotein, a cholesterol transport-related protein, or a metallothionein protein.

Table 1 shows the type of apolipoproteins according to the present invention, organs synthesizing them, and primary parts for localization of them.

**[Table 1]**

| Apolipoprotein | Synthesizing organ | Primary localization |
|---|---|---|
| ApoA-I | liver, small intestine | HDL, chylomicron |
| ApoA-II | liver, small intestine | HDL |
| ApoA-IV | liver, small intestine | chylomicron |
| ApoB-100 | liver | VLDL, LDL |
| ApoB-48 | small intestine | chylomicron |
| ApoC-I | liver | HDL, chylomicron, VLDL |
| ApoC-II | | |
| ApoC-III | | |
| ApoD | liver, small intestine | HDL, VLDL, LDL |
| ApoE | liver | chylomicron, VLDL, LDL, HDL |

| | | |
|---|---|---|
| HDL: high-density lipoprotein, VLDL: very-low-density lipoprotein, LDL: low-density lipoprotein | | |

Apolipoprotein is a protein specifically present on plasma lipoprotein.

Many common structures are found for different apolipoproteins, and the origins are suspected to be identical from observation of apolipoprotein genes. While there are several types of apolipoprotein, the apolipoproteins listed in Table 1 are family proteins similar in the synthesizing organ and localization.

Table 2 shows the type of cholesterol transport-related proteins according to the present invention, expressing tissues for them, functions of them, and change in expression of them by AD.

**[Table 2]**

| Cholesterol transport-related protein | Expressing tissue | Function | Change by AD |
|---|---|---|---|
| IFITM1 | ubiquitous | inhibition of cholesterol transport | increased RNA expression in postmortem brain of AD patient |
| IFITM2 | ubiquitous | inhibition of cholesterol transport | increased RNA expression in postmortem brain of AD patient |
| IFITM3 | ubiquitous | inhibition of cholesterol transport | increased RNA expression in postmortem brain of AD patient |
| NPC1 | ubiquitous | cholesterol transport | increased RNA expression in postmortem brain of AD patient |
| NPC2 | ubiquitous | cholesterol transport | |
| NPC1L1 | gastrointestinal tract | cholesterol absorption | |

IFITM2 or IFITM3 (herein, also referred to as "IFITM2/3") is an intracellular cholesterol transport-related protein expressed in the cell membrane and endoplasmic reticulum membrane, and in addition to them three isoforms are present in humans (IFITM-1, 5 and 10). Expression of IFITM1, 2, and 3 is facilitated by interferon γ (IFNγ) induced by viral infection. IFITM1, 2, and 3 are reported to antagonize a cholesterol transporter on the endoplasmic reticulum to inhibit cholesterol transport when the expression is facilitated. It has been reported that RNA expression analysis for postmortem brains of AD patients found enhanced expression of IFITM1, 2, and 3 (Molecular Neurodegeneration (2009) 4(5): 1-14, and IUBMB Life (2004) 56(6): 349-354). Thus, IFITM1, 2, and 3 have common features regarding expression sites and tendencies in change in expression by IFNγ and AD. On the other hand, IFITM5 and 10 are not induced by IFNγ, and the functions of them are currently unknown (J. Biol. Chem. (2015) 290: 25946-25959).

NPC1 is a causal gene for Niemann-Pick Disease type C, and known to be involved in cholesterol transport. It has been reported that RNA expression analysis for postmortem brains of AD patients found facilitated expression of NPC1 in the hippocampus (Biochimica et Biophysica Acta (2010) 1801: 831-838). In addition to NPC1, NPC2 is another causal gene for Niemann-Pick Disease type C. NPC1 and NPC2 are both expressed ubiquitously, and each has a function of intracellular cholesterol transport (Neurobiology of Disease (2014) 72: 37-47). Thus, NPC1 and NPC2 proteins have common features regarding expression sites and functions. Regarding NPC1, NPC1-L1 has 42% amino acid homology to human NPC1 (Genebank Accession No. AF002020).

MT is known as a protein to chelate heavy metal such as zinc, copper, and cadmium. In humans, nine isoforms of MT, namely, MT-1A, MT-1B, MT-1E, MT-1F, MT-1G, MT-1H, MT-1X, MT-2A, and MT-3, are known to be present. Table 3 shows isoforms of MT according to the present invention and expressing tissues for them.

**[Table 3]**

| MT | Expressing tissue |
|---|---|
| MT-1A | Ubiquitous |
| MT-1B | Ubiquitous |
| MT-1E | Ubiquitous |
| MT-1F | Ubiquitous |
| MT-1G | Ubiquitous |
| MT-1H | Ubiquitous |
| MT-1X | Ubiquitous |
| MT-2A | Ubiquitous |
| MT-3 | Brain |

In an embodiment, the urine biomarker is at least one urine protein (e.g., one urine protein, or combination of two, three, or more urine proteins) selected from the group consisting of ApoA-I, ApoA-II, ApoA-IV, ApoB-100, ApoB-48, ApoC-I, ApoC-II, ApoC-III, ApoD, ApoE, IFITM1, IFITM2, IFITM3, NPC1, NPC2, NPC1L1, and MT, or at least two urine proteins (e.g., combination of two, three, or more urine proteins) selected from the group described above.

In an embodiment, the urine biomarker is at least one urine protein, for example, one urine protein or combination of two, three, or more urine proteins, selected from the group consisting of ApoA-I, ApoB-100, ApoC-I, ApoD, ApoE, IFITM2, IFITM3, NPC1, and MT. In another embodiment, the urine biomarker is at least one urine protein, for example, one urine protein or combination of two, three, or more urine proteins, selected from the group consisting of ApoA-I, ApoB-100, ApoC-I, ApoD, ApoE, IFITM2, and IFITM3.

In an embodiment, the urine biomarker is at least one urine protein (e.g., one urine protein, or combination of two, three, or more urine proteins) selected from the group consisting of ApoA-I, ApoA-II, ApoA-IV, ApoB-100, ApoB-48, ApoC-I, ApoC-II, ApoC-III, ApoD, and ApoE, or at least two urine proteins (e.g., combination of two, three, or more urine proteins) selected from the group described above.

In an embodiment, the urine biomarker is at least one urine protein (e.g., one urine protein, or combination of two, three, or more urine proteins) selected from the group consisting of IFITM1, IFITM2, IFITM3, NPC1, NPC2, and NPC1L1, or at least two urine proteins (e.g., combination of two, three, or more urine proteins) selected from the group described above.

In an embodiment, the urine biomarker is at least one urine protein (e.g., one urine protein, or combination of two, three, or more urine proteins) selected from the group consisting of MT-1A, MT-1B, MT-1E, MT-1F, MT-1G, MT-1H, MT-1X, MT-2A, and MT-3, or at least two urine proteins (e.g., combination of two, three, or more urine proteins) selected from the group described above.

The "amount of a urine biomarker" is, for example, the content or concentration of the urine biomarker in a urine sample. Such values are measured by using a method capable of quantitatively measuring protein or partial peptide. Examples of such methods include, but are not limited to, ELISA utilizing or in combination with antigen-antibody reaction, Western blotting, mass spectrometry, and flow cytometry.

In another example, the amount of a urine biomarker may be a measure value (relative value) of a parameter obtained in a measurement method. In the case that fluorescence ELISA is used as the measurement method, as an example, the amount of a urine biomarker may be fluorescence intensity obtained from fluorescence ELISA. In measuring the amount of a urine biomarker in a urine sample derived from urine collected from a subject by using fluorescence ELISA, fluorescence ELISA measurement may be additionally performed for a standard sample containing a known concentration of the urine biomarker. In this case, comparison may be made in the step of determining, described later, between the fluorescence intensity of the urine sample and the fluorescence intensity of the standard sample (threshold).

In the case that a measurement method capable of counting particles such as flow cytometry is used, as another example, the count of molecules of a complex (e.g., extracellular vesicles) containing a urine biomarker may be used as the amount of the urine biomarker.

In the present invention, "determination" can be made in an automatic/systematic manner, without depending on decision by persons with expertise such as physicians and medical technologists. In an embodiment, determination is made in an automatic/systematic manner through comparison of the amount of a urine biomarker measured (measured value) with a "threshold" corresponding to the amount of the urine biomarker. For example, by using determination criteria pre-set so that a subject is determined to suffer from AD or have a high risk of developing AD if the measured value is higher than the threshold, determination can be made based on the difference between the measured value and the threshold even by persons without expertise. Although this example describes the case that the measured value is higher than the threshold, determination is not limited to such a manner. In accordance with the type of a urine biomarker, a subject may be determined to suffer from AD or have a high risk of developing AD if the measured value is lower than the threshold.

A "threshold" corresponding to the amount of a urine biomarker is a value set to determine whether a subject suffers from AD or has a high risk of developing AD based on the amount of the urine biomarker. In an example, such a threshold is a value to discriminate an AD group and a non-AD group based on the amount of a urine biomarker (diagnostic threshold). In setting the threshold, for example, the amounts of a urine biomarker with respect to an AD group and the amounts of a urine biomarker with respect to a non-AD group are measured, and the false-negative rate, false-positive rate, cost, and prevalence rate are considered. An ROC (Receiver Operator Characteristic Curve) can be used to set the threshold.

In another example, such a threshold may be a value to determine based on the amount of a urine biomarker whether a high risk of developing AD is expected and a certain support is required from the viewpoint of preventive medicine (a threshold with respect to preventive medicine). The threshold is set, for example, from relation between the amounts of a specific urine biomarker and AD incidence rates, the relation found in a cohort study. In another example, such a threshold may be empirically set.

In the case that one urine biomarker is used, as an example, one threshold may be set for the urine biomarker. In the case that combination of two or more urine biomarkers is used, as another example, one threshold may be set for each urine biomarker, and two thresholds in total may be used. In determination in this example, for example, a subject is determined to suffer from AD or have a high risk of developing AD in any one of the case that the amount of one urine biomarker (first measured value) is higher than a threshold corresponding to the amount of the urine biomarker (first threshold), and the case that the amount of the other urine biomarker (second measured value) is higher than a threshold corresponding to the amount of the urine biomarker (second threshold).

In the case that combination of two or more urine biomarkers is used, as another example, one threshold may be set for the combination. In determination in the case that the two or more urine biomarkers are co-localized in one urine protein-containing complex, a subject can be determined to suffer from AD or have a high risk of developing AD if the amount of the urine protein-containing complex including the two or more urine biomarkers co-localized therein is higher than a threshold corresponding to the amount of the urine protein-containing complex. In this case, the amount of urine biomarkers has the same meaning as the amount of the urine protein-containing complex including at least two urine biomarkers co-localized therein (e.g., the number of molecules of the complex), and the threshold corresponding to the amount of urine biomarkers has the same meaning as a threshold corresponding to the amount of the urine protein-containing complex (e.g., the number of molecules of the complex).

Although the above examples describe values for which thresholds are set in advance, the method for assisting diagnosis according to the present invention is not limited to such cases. For example, such a threshold may be the amount of a urine biomarker obtained in measurement for a standard reagent containing a predetermined concentration of the urine biomarker. The concentration of a urine biomarker in a standard reagent may be a concentration corresponding to any of the above diagnostic thresholds.

The term "AD group" refers to a group of subjects suffering from AD. The term "non-AD group" refers to a group of subjects not suffering from AD. The non-AD group may be, for example, a healthy subject group, a group of patients with another type of dementia (e.g., vascular dementia), or a group of patients with complication frequently found for AD (e.g., diabetes mellitus, heart disease). The term "healthy subject group" refers to a group of subjects selected out of healthy subjects with certain exclusion criteria. Such certain exclusion criteria may include no finding of signs associated with dementia. The scale of a group is appropriately set by those skilled in the art considering factors including the sensitivity, specificity, cost, and so forth of diagnosis.

Each of the AD group and the non-AD group may be classified into subgroups, for example, based on features (e.g., age, sex, and pathological condition) of subjects. In this case, a threshold is set so as to discriminate the AD group and the non-AD group for each subgroup. For example, the AD group and the non-AD group are each classified into subgroups based on age of subjects (e.g., "18 years old to younger than 65 years old" and "65 years old or older"). In this example, a threshold may be set so as to discriminate the AD group and the non-AD group for each subgroup (e.g., a juvenile AD group and a juvenile non-AD group).

Although determination is made on whether a subject "suffers from AD or has a high risk of developing AD" in the above examples, the method for assisting diagnosis according to the present invention is not limited to such a manner of determination, and determination to be made may be appropriately set. In an example, determination may be made on whether "a subject has a high probability of suffering from AD" or whether "a urine sample is derived from an AD patient".

In another example, determination to be made may be appropriately set in accordance with the pathological condition of a subject for measurement. In the case of a subject who is a patient having already been diagnosed with 1) mild cognitive impairment or 2) dementia in another clinical diagnosis method, for example, determination may be made on whether the subject suffers from 1) mild cognitive impairment caused by AD or 2) dementia caused by AD.

In the case of a subject for whom no sign of dementia is found, determination may be made on whether the subject has a risk of developing AD or is recommended to undergo an additional examination. Regarding a threshold corresponding to the amount of a urine biomarker, in this case, the amount of the urine biomarker is measured for a healthy subject group, and the upper or lower limit of the median 95% confidence interval (reference range) for the measured values or (mean ± 2 × standard deviation) for the measured values may be used as the threshold. In an example, such a threshold may be the upper limit of a reference range for the amount of the corresponding urine biomarker.

In an embodiment of the first aspect of the present invention, the step of measuring the amount of a biomarker in a urine sample derived from urine collected from a subject includes forming a conjugate of the urine biomarker with a detection reagent for the urine biomarker and detecting a signal reflecting the amount of the urine biomarker derived from the conjugate. In another embodiment, the step of measuring further includes calculating the amount of the urine biomarker from the signal detected.

The "detection reagent" for a urine biomarker contains a "probe" capable of specifically binding to a urine biomarker of interest. Examples of the probe include antibodies and compounds for a urine biomarker. Examples of such antibodies include, but are not limited to, intact antibodies (e.g., monoclonal antibodies, polyclonal antibodies), antibody fragments (e.g., Fab, Fab', F(ab')₂), and synthesized antibodies (e.g., single-chain antibodies (scFv), chimeric antibodies, humanized antibodies). Such an antibody can be prepared by using a known method such as an immunological technique, phage display, and ribosome display. A commercially available antibody may be directly used as a probe. Examples of the compound include substances such as aptamers capable of specifically binding to a urine biomarker.

The probe may be present as a free form, or immobilized on a carrier such as beads. Examples of the carrier include beads and a plate. The material of the beads or plate is not limited, and may be, for example, resin. The beads may be, but are not limited to, metal particles, resin particles, or semiconductor particles. The beads may be magnetized. The beads may contain a fluorescent substance, and the beads themselves may be fluorescent objects, for example, quantum dots. The plate may be, but is not limited to, a microtiter plate made of resin and including a bottom surface made of resin or glass.

When a urine biomarker in a urine sample and a detection reagent containing a probe for the urine biomarker are left under conditions allowing them to come into contact, a "conjugate" of the urine biomarker with the detection reagent is formed. Formation of the conjugate is achieved, for example, in a solution environment. In the case that an antibody is immobilized on a microtiter plate, as an example, a urine sample in the form of solution is added to the microtiter plate, allowing the antibody immobilized and the urine biomarker (antigen) in the urine sample to come into contact in a solution environment. Antigen-antibody reaction of them can form an immune complex. After a conjugate is formed, the conjugate may be separated from the urine biomarker or the detection reagent left unreacted (B/F separation).

Formation of a conjugate of the urine biomarker with the detection reagent can be accelerated through concentration or purification of the urine biomarker in urine. In an example, the urine sample may have been enriched to increase the concentration or content of urine protein or a urine protein-containing complex in urine.

In "enrichment" treatment for urine protein, for example, any known method for concentrating or purifying protein or peptide can be used without any particular limitation. In "enrichment" treatment for a urine protein-containing complex, for example, any known method for concentrating or purifying vesicles of a lipid bilayer membrane such as extracellular vesicles can be used without any particular limitation. Examples of enrichment treatment for extracellular vesicles include, but are not limited to, centrifugation, microfiltration, and a surface antigen affinity method. In an example, "enrichment" treatment for urine protein in a free form includes centrifuging urine collected from a subject followed by collecting a fraction (e.g., the supernatant) containing urine protein in a free form. In another example, "enrichment" treatment for urine protein in a free form includes subjecting a urine sample enriched with a urine protein-containing complex to extraction treatment, described later. The enrichment treatment for urine protein in a free form or present in a complex reduces contaminated substances including contaminated protein in a urine sample, and as a result the urine protein or the urine protein-containing complex can be partially purified.

In the case that a urine biomarker included in extracellular vesicles or present in lipid bilayer membranes thereof is to be measured, urine may be subjected to extraction treatment so that a detection reagent for the urine biomarker and the urine biomarker can come into contact or it becomes easier for them to come into contact. The extraction treatment refers to collecting the urine biomarker from the inside of lipid bilayer membranes of the extracellular vesicles, or from lipid bilayer membranes thereof. In the case that the urine biomarker is present on lipid bilayer membranes of the extracellular vesicles and the detection reagent can come into contact with the corresponding binding surface on the urine biomarker, no extraction treatment may be performed. Even in this case, the extraction treatment may be performed to accelerate the formation of the conjugate of the urine biomarker with the detection reagent.

For the "extraction" treatment for a urine biomarker from a urine protein-containing complex (e.g., extracellular vesicles), any known method for collecting protein or partial peptide from vesicles of lipid bilayer membranes such as extracellular vesicles can be used without any particular limitation. Examples of the extraction treatment include, but are not limited to, a process of extracting with alkaline solution (hereinafter, referred to as "alkali extraction process"), a process of extracting through freezing and thawing (hereinafter, referred to as "freeze-thaw extraction process"), and a process of extracting with surfactant (hereinafter, referred to as "surfactant extraction process").

While a urine biomarker extracted from a urine protein-containing complex (e.g., extracellular vesicles) is typically in a free form in the urine sample, the urine biomarker may be immobilized on a carrier such as a plate.

The urine sample may have been subjected to enrichment treatment and extraction treatment for a urine protein-containing complex to accelerate the formation of a conjugate of a urine biomarker with a detection reagent for the urine biomarker, for example. In an embodiment, the step of preparing the urine sample by using urine collected from a subject is included, and the step of preparing the urine sample may include enrichment treatment and/or extraction treatment for a urine protein-containing complex (e.g., extracellular vesicles). In the case that no extraction treatment is performed, the urine sample can contain a urine protein-containing complex (e.g., extracellular vesicles) containing a urine biomarker derived from the urine. In an embodiment in this case, the urine biomarker is preferably at least one urine protein, for example, one urine protein or combination of two, three, or more urine proteins selected from the group consisting of ApoA-I, ApoA-II, ApoA-IV, ApoB-100, ApoB-48, ApoC-I, ApoC-II, ApoC-III, ApoD, and ApoE.

In the case that co-localization of two or more urine biomarkers in a urine protein-containing complex (e.g., extracellular vesicles) is used as an index, enrichment treatment may be performed for extracellular vesicles, though extraction treatment is not performed in typical cases.

The detection reagent may further contain a "labeling substance" to emit a signal, in addition to a probe. Examples of labeling substances include fluorescent substances, radioactive substances, and enzymes. Any substance of known fluorescent substances, radioactive substances, and enzymes can be used without any particular limitation, which are commercially available. Fluorescent substances and enzymes can be produced, for example, by using a known method. In the case that an enzyme is used as a labeling substance, the detection reagent contains a substrate for the enzyme. Examples of the substrate include chromogenic substrates, chemifluorescent substrates, and chemiluminescent substrates.

The labeling substance may be bound to a probe in advance to exist as a labeled probe. In labeling, the labeling substance may be directly bound to a probe, or indirectly bound to a probe via at least one additional substance. In the case that biotin has been bound to a probe for a urine biomarker, the labeling substance is bound to an avidin (e.g., avidin, streptavidin). In this case, the labeling substance is indirectly bound to the probe via binding between biotin and the avidin.

In the case that the detection reagent contains a labeling substance, the labeling substance can emit a signal reflecting the amount of a urine biomarker from a conjugate of the urine biomarker with the detection reagent. If the conjugate is formed in a manner depending on (e.g., in proportion to) the amount of the urine biomarker in a urine sample, for example, the intensity of the signal can reflect the amount of the urine biomarker in the urine sample. Based on the signal intensity (relative value) obtained, determination can be made on whether a provider of the urine sample measured (subject) suffers from AD or has a high risk of developing AD. Alternatively, the concentration or content of the urine biomarker in the urine sample is calculated from signal intensity obtained from the urine sample by using signal intensity obtained from a standard sample with a known concentration, and based on the calculated value determination can be made on whether a provider of the urine sample measured (subject) suffers from AD or has a high risk of developing AD.

Signal type changes depending on the type of the labeling substance used, for example. The detection method and the detector for signals are appropriately set by those skilled in the art in accordance with the signal type. In the case that a fluorescent substance is used as a labeling substance, for example, a fluorescent signal may be detected. The fluorescent signal can be detected by using a known detector such as a fluorescence spectrometer, a microscope, a flow cytometer, and a microplate reader. In the case that a radioactive substance is used as a labeling substance, a radioactive signal may be detected. The radioactive signal can be detected by using a known detector such as a scintillation counter.

In the case that an enzyme is used as a labeling substance, a color signal, a fluorescent signal, or a luminescent signal may be detected, the signal depending on a substrate for the enzyme. In the case that an enzyme and a chromogenic substrate are used, for example, where coloring or color change is detected as a signal, the signal can be detected by using a known detector or through visual observation. In the case that an enzyme and a chemifluorescent substrate or a chemiluminescent substrate is used, for example, where fluorescence or luminescence is detected as a signal, the signal can be detected by using a known detector.

The amount of urine protein may be measured through mass spectrometry utilizing antigen-antibody reaction (also referred to as immuno-MS or mass-linked immuno-selective analysis (MALISA)). In this case, the signal is in a mass-to-charge ratio, and a labeling substance is not necessarily required. In the case that mass spectrometry is used for the measurement method, measurement can be achieved with discriminating post-translational modification such as isoforms which may be formed as a result of single amino acid substitution and glycosylation.

Those skilled in the art could appropriately set the detection reagent according to the present invention in accordance with the measurement method for a urine biomarker, for example. In the case that sandwich ELISA is used as the measurement method, as an example, the detection reagent contains, for example, a first antibody for a urine biomarker immobilized on a microtiter plate; a second antibody which binds to an epitope differing from an epitope to which the first antibody binds on the urine biomarker; a third antibody labeled with an enzyme for the second antibody; and a substrate for the enzyme. The embodiment of ELISA is not limited to sandwich ELISA, and other embodiments (a direct adsorption method, a competitive method (direct competitive ELISA)) can be used.

Regarding an embodiment, a method for assisting diagnosis by using ELISA with direct immunofluorescence assay as the measurement method will be described. The detection reagent contains, for example, an antibody for a urine biomarker, the antibody labeled in advance with a fluorescent substance (hereinafter, also referred to as "fluorescently labeled antibody"). The standard sample contains a predetermined concentration (threshold) of the urine biomarker purified in a free form.

A urine sample in the form of solution is aliquoted into a microtiter plate, and the urine biomarker in the urine sample is allowed to be adsorbed on the microtiter plate. The urine sample is removed (B/F separation), and blocking treatment is performed. A solution containing a fluorescently labeled antibody is aliquoted into the microtiter plate to form an immune complex of the urine biomarker adsorbed (immobilized) with the fluorescently labeled antibody. The sample containing the complex contained in the microtiter plate is irradiated with excitation light, and a fluorescent signal derived from the immune complex is measured. The fluorescent signal measured is compared with a fluorescent signal similarly measured for the standard sample to determine whether the subject suffers from AD or has a high risk of developing AD.

Although a fluorescent substance is used as a labeling substance in the described embodiment, the labeling substance is not limited thereto, and a radioactive substance or an enzyme may be used.

In place of the microtiter plate in ELISA, a microarray (microchip) can be used in which antibodies or aptamers for a plurality of urine biomarkers are immobilized in alignment (array) on a carrier (substrate).

In the case that the detection reagent contains probes for two or more urine biomarkers, the method for assisting diagnosis according to the present invention may be diagnosis to make determination by comparing the amounts of two or more urine biomarkers (two or more measured values) with two or more respective thresholds. In this case, a subject may be determined to suffer from AD or have a high risk of developing AD if one of the measured values is higher than the corresponding threshold. The method for assisting diagnosis using at least two urine biomarkers can enable diagnosis with higher sensitivity than the method for assisting diagnosis using one urine biomarker.

The method for assisting diagnosis according to the present invention may use co-localization of two or more urine biomarkers in a urine protein-containing complex as an index. In this case, a sample enriched with a urine protein-containing complex derived from urine collected from a subject may be used as a urine sample, though the urine sample is not limited thereto. This embodiment enables AD diagnosis with high sensitivity, as demonstrated later in Examples. In addition, this embodiment can provide a method for assisting diagnosis of heart disease with high sensitivity.

Accordingly, another aspect of the present invention provides a method for assisting diagnosis of AD and/or heart disease based on co-localization of at least two urine biomarkers in a urine protein-containing complex.

An embodiment of the present aspect provides a method for assisting diagnosis of AD and/or heart disease, the method including the steps of: measuring the amount of a urine protein-containing complex containing at least two urine biomarkers in a urine sample derived from urine collected from a subject; and determining whether the subject suffers from Alzheimer's disease and/or heart disease or has a high risk of developing AD and/or heart disease, wherein the urine biomarkers are combination of at least two urine proteins, for example, combination of two, three, or more urine proteins selected from the group consisting of ApoA-I, ApoA-II, ApoA-IV, ApoB-100, ApoB-48, ApoC-I, ApoC-II, ApoC-III, ApoD, ApoE, IFITM1, IFITM2, IFITM3, NPC1, NPC2, NPC1L1, and MT.

In the embodiment of the present aspect, for example, ELISA and flow cytometry can be used as a detection method.

As an example, a case with sandwich ELISA will be described. The detection reagent contains, for example, a first probe for a first urine biomarker, the first probe immobilized on a plate, and a second probe for a second urine biomarker, the second probe labeled in advance with a standard substance. In this example, a urine sample possibly containing a urine protein-containing complex (including extracellular vesicles) derived from urine collected from a subject is aliquoted into the plate. This allows the urine protein-containing complex containing the first urine biomarker to be immobilized on the plate via the first probe. After B/F separation, the second probe is aliquoted into the plate. This allows the second probe to bind to the urine protein-containing complex containing the second urine biomarker. A three-membered conjugate including the first probe, the second probe, and the complex having the first and second urine biomarkers co-localized therein is immobilized on the plate. The amount of the three-membered conjugate is measured after B/F separation. By comparing the measured value with the threshold, determination can be made on whether the subject suffers from AD and/or heart disease.

As another example, a case with flow cytometry will be described. The detection reagent contains, for example, a first probe for a first urine biomarker, the first probe immobilized on a first quantum dot (first labeling substance), and a second probe for a second urine biomarker, the second probe immobilized on a second quantum dot (second labeling substance). In this example, the first and second quantum dots are excited at λ1, and the first quantum dot emits fluorescence with a peak wavelength of λ2 and the second quantum dot emits fluorescence with a peak wavelength of λ3. A urine sample possibly containing a urine protein-containing complex derived from urine collected from a subject is contacted with the detection reagent to form a three-membered conjugate in which the first probe and the second probe is bound to the complex having the first and second urine biomarker co-localized therein.

The three-membered conjugate doubly stained with the first probe and the second probe is introduced into a flow cytometer to measure a signal. The flow cytometer is an apparatus which irradiates a flow of fluid converged in a fluid mechanics sense with a beam (e.g., a laser beam) of a specific wavelength to acquire optical information derived from individual particles contained in the fluid and analyzes the physical and chemical characteristics of the individual particles based on the optical information. In this example, by irradiating a flux of fluid containing individual molecules of the conjugate with λ1 excitation light, fluorescence (peak wavelength: λ2 and λ3) can be measured from the individual molecules of the conjugate. The conjugate emitting light with peak wavelengths of λ2 and λ3 can be regarded as a urine protein-containing complex including the first and second urine biomarkers co-localized therein, and the molecules can be counted. By comparing the count value (measured value) with a threshold corresponding to the urine protein-containing complex, determination can be made on whether the subject suffers from AD and/or heart disease.

In an embodiment, the threshold is, for example a value suitable for discriminating a heart disease group and a non-heart disease (e.g., healthy subject) group (diagnostic threshold). In the step of determining, for example, a subject may be determined to suffer from heart disease or have a high risk of developing heart disease if the measured value is higher than the threshold. This embodiment includes the step of determining whether the subject suffers from heart disease or has a high risk of developing heart disease, in place of the step of determining whether the subject suffers from AD or has a high risk of developing AD. Accordingly, another aspect of the present invention provides a method for assisting diagnosis of heart disease based on co-localization of at least two urine biomarkers in a urine protein-containing complex. In an embodiment, the heart disease in the embodiment is cardiac hypertrophy.

In an embodiment, the urine biomarkers are combination of at least two urine proteins, for example, two, three, or more urine proteins selected from the group consisting of ApoA-I, ApoB-100, ApoC-I, ApoD, ApoE, IFITM2, IFITM3, NPC1, and MT. In an embodiment, the urine biomarkers are preferably combination of at least two urine proteins, for example, two, three, or more urine proteins selected from the group consisting of ApoA-I, ApoA-II, ApoA-IV, ApoB-100, ApoB-48, ApoC-I, ApoC-II, ApoC-III, ApoD, and ApoE.

A second aspect of the present invention provides a detection reagent for use in the method for assisting diagnosis according to the first aspect. The detection reagent contains a probe for any of the urine biomarkers described herein, and, optionally, further contains a labeling substance. The probe in the detection reagent may be in the form of liquid or solid, or present in a free form, or immobilized on a carrier such as a microtiter plate or beads, though the form of the probe is not limited thereto. The labeling substance in the detection reagent may be present singly, or be bound in advance to the probe for labeling. The features described herein with respect to elements including a probe and a labeling substance are also applied to the corresponding elements according to the present aspect.

A third aspect of the present invention provides a diagnosis kit for use in the method for assisting diagnosis according to the first aspect. The diagnosis kit includes the detection reagent according to the second aspect, and may further include reagents for buffering, for washing, for blocking, and for quenching each in the form of liquid or in the form of solid, and a carrier such as a plate and beads, though such additional components are not limited thereto. The features described herein with respect to elements including a probe, a labeling substance, a carrier, a threshold, and determination are also applied to the corresponding elements according to the present aspect.

The diagnosis kit may include a standard sample containing a predetermined concentration or predetermined amount of a standard substance such as a urine biomarker. The reagents in the diagnosis kit may be separately contained in different reagent containers, and if coexistence of reagents does not cause any problem in measuring a urine biomarker, they may be contained in the same reagent container. The diagnosis kit may further include an accompanying document. The accompanying document may describe information including how to use the reagents, how to use the standard reagent, how to prepare a standard curve, how to calculate the amount of a urine biomarker from the standard curve, and determination criteria. In an example of determination criteria to be described, a subject is determined to suffer from AD or have a high risk of developing AD if the calculated value of a urine biomarker in the urine sample is higher or lower than a threshold corresponding to the amount of the urine biomarker.

A fourth aspect of the present invention provides a diagnosis system for AD. The diagnosis system includes a determination section and an indication section, where the determination section compares the amount of a urine biomarker in a urine sample derived from urine collected from a subject with a threshold corresponding to the amount of the urine biomarker with respect to AD and determines whether the subject suffers from AD, and the indication section indicates a determination result from the determination section. The features described herein with respect to elements including a urine biomarker, the amount of the urine biomarker, a threshold corresponding to the amount of the urine biomarker, and determination are also applied to the corresponding elements according to the present aspect.

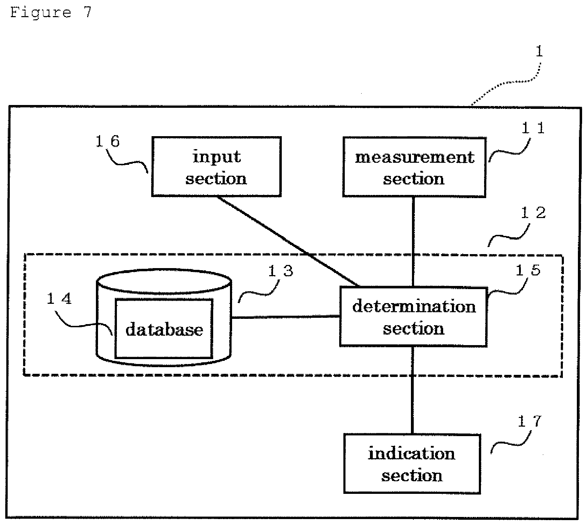

Figure 7 shows a block diagram illustrating the schematic configuration of a diagnosis system 1 according to an embodiment. The diagnosis system 1 includes: a measurement section 11; a controller 12; an input section 16; and an indication section 17. The controller 12 includes a storage 13 storing a database 14; and a determination section 15.

The measurement section 11 is configured with a device for use in a method capable of quantitatively measuring protein. The measurement section 11 may be an imaging device to take an image with a microplate reader for use in ELISA or a CCD, an imaging device for use in a method utilizing Western blotting or a microarray (microchip), a mass spectrometer for use in mass spectrometry, or a flow cytometer for use in flow cytometry. The measurement section 11 has a function to output measurement data to the controller 12.

The controller 12 includes: a processing circuit corresponding to a processor such as a CPU; a memory (main memory); and the storage 13. For example, a computer can be used for the controller 12. The processor of the controller 12 executes computer programs loaded into the memory.

The storage 13 is an auxiliary storage, and may be, for example, a hard disk drive (HDD) or a solid state drive (SSD). The storage 13 stores, for example, computer programs. The computer programs are loaded into the memory and executed by the processor. The computer programs include an operating system and application programs. The application programs include a threshold acquisition program to acquire a threshold stored in the database 14 for the amount of a urine biomarker, a determination program to activate determination function, described later, and an indication program to indicate determination results or the like. In this example, the storage 13 stores the database 14.

The database 14 stores threshold data corresponding to the amount of a urine biomarker. The threshold data include data of thresholds corresponding to various urine biomarkers described herein to discriminate an AD group and a non-AD group. The threshold data may include data of thresholds corresponding to the various urine biomarkers to discriminate an AD group and a non-AD group in a subgroup obtained by classifying subjects based on their features (e.g., age, sex, and pathological condition). The threshold data may include data of thresholds regarding the upper or lower limit of a reference range corresponding to each urine biomarker in a healthy subject group. The database 14 may be further storing measurement data or the like from the measurement section 11. Although the threshold data are here described in the context of AD as a target disease, the diagnosis system according to the present invention is not limited thereto, and the threshold data include, for example, data of thresholds corresponding to various urine biomarkers described herein with respect to heart disease to discriminate a heart disease group and a non-heart disease group. Provided as an embodiment is the diagnosis system wherein the determination section further determines whether the subject suffers from heart disease or has a high risk of developing heart disease by comparing, with a threshold with respect to heart disease, the amount of a urine protein-containing complex including at least two urine biomarkers co-localized therein in a urine sample. Provided as another embodiment is the diagnosis system wherein the determination section determines whether the subject suffers from heart disease or has a high risk of developing heart disease by comparing, with a threshold with respect to heart disease, in place of a threshold with respect to AD, the amount of a urine protein-containing complex including at least two urine biomarkers co-localized therein in a urine sample.

In the case that the database 14 is stored in a storage medium such as a magnetic disk, an optical disk, a magneto-optical disk, and a flash memory, the storage 13 may be configured with: a drive device to read/write information from/in the storage medium; and the storage medium.

The determination section 15 has a function to compare an amount of a urine biomarker in a urine sample derived from urine collected from a subject with a threshold corresponding to the amount of the urine biomarker with respect to AD and/or heart disease, thereby determining whether the subject suffers from AD and/or heart disease or has a high risk of developing AD and/or heart disease. The function of the determination section 15 is achieved through a process such that the processing circuit including the processor of the controller 12 executes application programs including the above-mentioned threshold acquisition program and determination program loaded into the memory of the controller 12.

The input section 16 is configured with an instrument or device by which a user inputs necessary information (identification information) and instruction into the controller 12. The input section 16 may be, for example, a keyboard, a mouse, or a voice recognition device. The identification information to be input by a user includes, for example, information on the type of a urine biomarker, the type of the disease, and the sex and age of a subject.

The indication section 17 is configured with a device capable of allowing a user to perceive a determination result or the like from the determination section 15, and may be, for example, a display, an indicator light, a speaker, or a printer.

In Figure 7, a solid line connecting the measurement section 11 and the determination section 15 is configured with a transmitter /receiver including an interface to activate sending/receiving of data and signals between the elements through wired or wireless connection. The same is applied to other solid lines connecting elements in Figure 7.

Although the database 14 is stored in the storage 13 in the inside of the controller 12 in the above example, the diagnosis system according to the present invention is not limited to such configuration. For example, the database 14 may be stored in a storage present outside of the diagnosis system. Such a storage may be, for example, configured with the entire or part of a storage medium such as an optical disk, and may be provided to a server connected to the diagnosis system according to the present invention via a network.

Although the diagnosis system includes the measurement section 11 in the above example, the diagnosis system according to the present invention is not limited to such configuration. For example, measurement data obtained by using a measurement apparatus present outside of the diagnosis system may be read by the controller 12 and stored, for example, in the storage 13. Alternatively, measurement data obtained in the outside may be stored in a storage present outside of the diagnosis system, as described above.

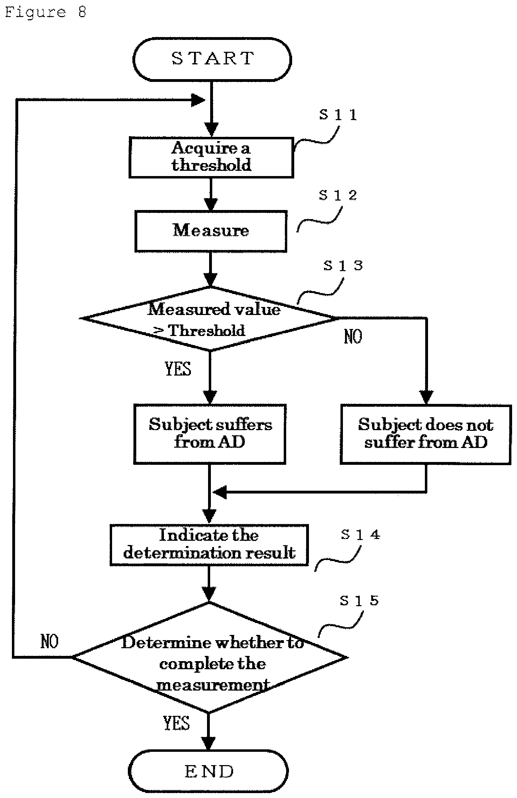

The flow of the diagnosis system for AD according to the present invention (Embodiment 1) will be described with reference to Figures 7 and 8.

A user inputs instruction to the controller 12 and necessary information (identification information) by using the input section 16. In this example, the identification information includes the following information: two measurement samples (samples 1 and 2); target disease: AD; type of urine biomarker: ApoC-I for all cases; and age of subjects: 60 years old for sample 1, 70 years old for sample 2. When instruction and identification information are input by a user via the input section 16, the processor of the controller 12 acquires a threshold (S11).

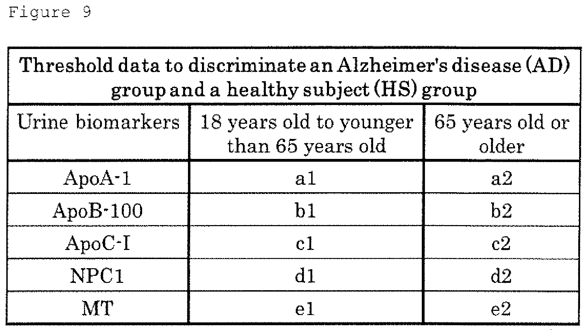

In the step of acquiring a threshold (S11), the processor executes the threshold acquisition program loaded from the storage 13 into the memory. The threshold acquisition program executed refers to the identification information input by the user, and acquires the corresponding threshold from threshold data stored in the database 14. Figure 9 shows an example of threshold data stored in the database 14. In the threshold data, an AD group and a non-AD group are classified into subgroups based on age of subjects ("18 years old to younger than 65 years old" and "65 years old or older"). The threshold data include thresholds (a1 to e1 and a2 to e2) corresponding to five urine biomarkers (ApoA-1, ApoB-100, ApoC-I, NPC1, and MT) to discriminate an Alzheimer's disease (AD) group and a healthy subject (HS) group for each subgroup.

In this example, the threshold acquisition program executed first acquires the threshold c1 in accordance with the identification information on the sample 1 (target disease: AD, urine biomarker: ApoC-I, age: 60 years old).

When the threshold is acquired, the processor of the controller 12 sends a measurement initiation signal to the measurement section 11. On receiving the measurement initiation signal from the controller 12, the measurement section 11 initiates measurement (S12). In the step of measuring (S12), the urine sample (1) derived from urine collected from the subject is subjected to measurement with the measurement section 11, and data (measurement data (1)) on the amount of the urine biomarker (ApoC-I) contained in the urine sample are generated. The measurement section 11 sends the generated measurement data (1) to the controller 12. The generated measurement data (1) includes a value (measured value) of the amount of the urine biomarker (ApoC-I) for the subject.

On receiving the measurement data (1) from the measurement section 11, the controller 12 makes determination (S13). In the step of determining (S13), the processor of the controller 12 executes the determination program loaded from the storage 13 into the memory. The determination program executed compares a measured value included in the measurement data (1) received from the measurement section 11 with the threshold c1 acquired in the step of acquiring a threshold. The determination program determines that the subject suffers from AD (YES) if the measured value is higher than the threshold c1, and that the subject does not suffer from AD (NO) if the measured value is equal to or lower than the threshold c1, and determination data (1) including the determination result are generated.

When the determination data (1) are generated, the processor of the controller 12 indicates the determination result on the indication section 17 (S14). In the step of indicating a determination result (S14), the processor executes the indication program loaded from the storage 13 into the memory, and outputs video signals corresponding to the determination result onto the indication section 17 (e.g., a display). The display indicates the determination result based on the video signals input.

The processor of the controller 12 determines whether to complete measurement (S15). Since measurement for the sample (2) of the two samples (1 and 2) has not been completed at this point, a determination result of (NO) is presented in the step of determining whether to complete measurement (S15), and S11 to S14 are repeated.

Specifically, the threshold c2 is acquired from the threshold data in accordance with the identification information on the sample 2 (target disease: AD, urine biomarker: ApoC-I, age: 70 years old) in the step of acquiring a threshold (S11). In the step of measuring (S12), measurement data (2) on the amount of the urine biomarker (ApoC-I) in the urine sample (2) derived from urine collected from the subject are generated in the measurement section 11. In the step of determining (S13), a measured value included in the measurement data (2) is compared with the threshold c2 acquired in the step of acquiring a threshold (S11), and determination data (2) are generated. In the step of indicating a determination result (S14), the determination result is indicated on the indication section 17.

Thereafter, the processor of the controller 12 determines completion of measurement (YES), and measurement is completed.

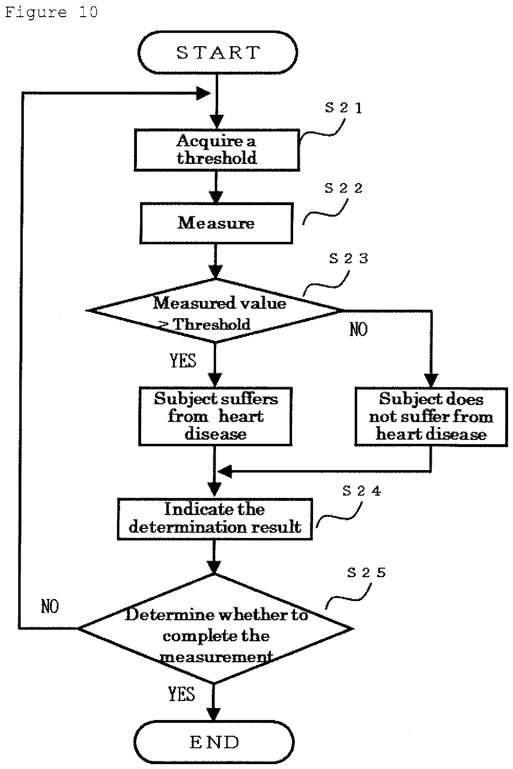

The flow of the diagnosis system for heart disease according to another embodiment (Embodiment 2) will be described with reference to Figures 7 and 10.

A user inputs instruction to the controller 12 and necessary information (identification information) by using the input section 16. In this example, the identification information includes the following information: two measurement samples (samples 1' and 2'); target disease: heart disease; type of urine biomarker: combination of ApoB and ApoC-I for all cases; age of subjects: 60 years old for sample 1', 70 years old for sample 2'. When instruction and identification information are input by a user via the input section 16, the processor of the controller 12 acquires a threshold (S21) .

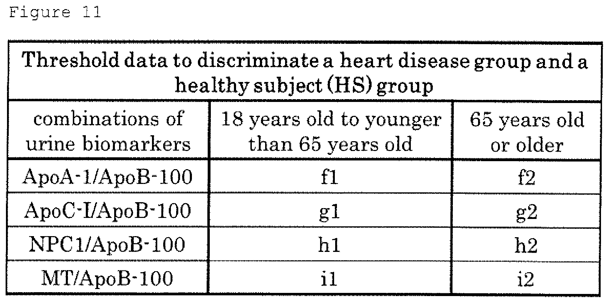

In the step of acquiring a threshold (S21), the processor executes the threshold acquisition program loaded from the storage 13 into the memory. The threshold acquisition program executed refers to the identification information input by the user, and acquires the corresponding threshold from threshold data stored in the database 14. Figure 11 shows an example of threshold data stored in the database 14. In the threshold data, a heart disease group and a non-heart disease group are classified into subgroups based on age of subjects ("18 years old to younger than 65 years old" and "65 years old or older"). The threshold data include thresholds (f1 to i1 and f2 to i2) for four combinations of urine biomarkers to discriminate a heart disease group and a healthy subject (HS) group for each subgroup.

In this example, the threshold acquisition program executed first acquires the threshold g1 in accordance with the identification information on the sample 1' (target disease: heart disease, urine biomarker: combination of ApoB and ApoC-I, age: 60 years old).

When the threshold is acquired, the processor of the controller 12 sends a measurement initiation signal to the measurement section 11. On receiving the measurement initiation signal from the controller 12, the measurement section 11 initiates measurement (S22). In the step of measuring (S22), the urine sample (1') derived from urine collected from the subject is subjected to measurement with the measurement section 11, and data (measurement data (1')) on the amount of the urine biomarkers (co-localized ApoB and ApoC-I) contained in the urine sample are generated. The measurement section 11 sends the measurement data (1') generated to the controller 12. The measurement data (1') generated includes a value (measured value) of the amount of the urine biomarkers (co-localized ApoB and ApoC-I) for the subject.

On receiving the measurement data (1') from the measurement section 11, the controller 12 makes determination (S23). In the step of determining (S23), the processor of the controller 12 executes the determination program loaded from the storage 13 into the memory. The determination program executed compares a measured value included in the measurement data (1') received from the measurement section 11 with the threshold g1 acquired in the step of acquiring a threshold. The determination program determines that the subject suffers from heart disease (YES) if the measured value is higher than the threshold g1, and that the subject does not suffer from heart disease (NO) if the measured value is equal to or lower than the threshold g1, and determination data (1') including the determination result are generated.

When the determination data (1') are generated, the processor of the controller 12 indicates the determination result on the indication section 17 (S24). In the step of indicating a determination result (S24), the processor executes the indication program loaded from the storage 13 into the memory, and outputs video signals corresponding to the determination result onto the indication section 17 (e.g., a display). The display indicates the determination result based on the video signals input.

The processor of the controller 12 determines whether to complete measurement (S25). Since measurement for the sample (2') of the two samples (1' and 2') has not been completed at this point, a determination result of (NO) is presented in the step of determining whether to complete measurement (S25), and S21 to S24 are repeated.

Specifically, the threshold g2 is acquired from the threshold data in accordance with the identification information on the sample 2' (target disease: heart disease, urine biomarker: combination of ApoB and ApoC-I, age: 70 years old) in the step of acquiring a threshold (S21). In the step of measuring (S22), measurement data (2') on the amount of the urine biomarkers (co-localized ApoB and ApoC-I) in the urine sample (2') derived from urine collected from the subject are generated in the measurement section 11. In the step of determining (S23), a measured value included in the measurement data (2') is compared with the threshold g2 acquired in the step of acquiring a threshold (S21), and determination data (2') are generated. In the step of indicating a determination result (S24), the determination result is indicated on the indication section 17.

Thereafter, the processor of the controller 12 determines completion of measurement (YES), and measurement is completed.

Although the step of acquiring a threshold (S11, S21) is performed prior to the step of measuring (S12, S22) in the above examples, the diagnosis system according to the present invention is not limited to such order. In the diagnosis system according to the present invention, for example, it is only needed that the step of acquiring a threshold (S11, S21) and the step of measuring (S12, S22) are performed before the step of determining (S13, S23) is performed. Accordingly, the step of acquiring a threshold (S11, S21) may be performed simultaneously with the step of measuring (S12, S22), or after the step of measuring (S12, S22).

Although S11 to S14/S21 to S24 are performed for one urine sample (sample 1, sample 1') and S11 to S14/S21 to S24 are repeated for the other urine sample (sample 2, sample 2') in the above examples, the diagnosis system according to the present invention is not limited to such order. For example, the diagnosis system according to the present invention may be such that thresholds for all urine samples to be determined are acquired at once in accordance with identification information input by a user (S11, S21), the amount of a urine biomarker is measured for all urine samples (S12, S22), determination is made for each of the urine samples on whether the subject suffers from AD/heart disease (S13, S23), and all the determination results may be indicated (S14, S24).

Although the step of indicating a determination result (S14, S24) is performed prior to the step of determining whether to complete measurement (S15, S25) in the above examples, the diagnosis system according to the present invention is not limited to such order. For example, the diagnosis system according to the present invention may be such that determination is made for all urine samples designated (S15, S25) in accordance with identification information input by a user, and after the completion the determination results are indicated (S14, S24) .

Although threshold data stored in advance in a database are used to acquire a threshold in the above examples, the diagnosis system according to the present invention is not limited to such configuration. For example, a user inputs information (identification information) on a standard sample (containing a known concentration of the urine biomarker ApoC-I, or a complex containing a known amount of combination of urine biomarkers). In the measurement section 11, measurement data (including measured values) on the amount of a urine biomarker(s) in the standard sample are generated (S12, S22). The measurement data generated are sent from the measurement section 11 to the controller 12, and stored in the storage 13. The processor of the controller 12 then executes the threshold acquisition program loaded into the memory (S11, S21). The threshold acquisition program executed acquires a measured value included in the measurement data obtained in the step of measuring (S12, S22) from the threshold data stored in the storage 13 in accordance with the identification information input by the user. The subsequent step of determining (S13, S23), step of indicating a determination result (S14, S24), and step of determining whether to complete measurement (S15, S25) may be performed as described above.

In the diagnosis system in another embodiment (Embodiment 3), a diagnostic flow for AD and a diagnostic flow for heart disease are performed. The flow of the diagnosis system to perform a diagnostic flow for AD and then a diagnostic flow for heart disease will be described with reference to Figures 7, 8, and 10.

A user inputs instruction to the controller 12 and necessary information (identification information) by using the input section 16. In this example, the identification information includes the following information: two measurement samples (samples 1" and 2"); target disease: AD for sample 1", heart disease for sample 2"; type of urine biomarker: ApoC-I for sample 1", combination of ApoB and ApoC-I for sample 2"; and age of subjects: 60 years old for sample 1", 70 years old for sample 2".

When instruction and identification information are input by a user via the input section 16, as described above, the processor of the controller 12 performs acquisition of a threshold (S11 in Figure 8, Figure 9 (c1)), measurement (S12 in Figure 8), comparison of a measured value with the threshold (S13 in Figure 8), indication of a determination result (S14 in Figure 8), and determination whether to complete measurement (S15 in Figure 8) for the sample 1". After the completion of measurement for the sample 1", the processor of the controller 12 performs acquisition of a threshold (S21 in Figure 10, Figure 11 (g2)), measurement (S22 in Figure 10), comparison of a measured value with the threshold (S23 in Figure 10), indication of a determination result (S24 in Figure 10), and determination whether to complete measurement (S25 in Figure 10) for the sample 2".

Although the diagnostic flow for AD is followed by the diagnostic flow for heart disease in the above example, the diagnosis system according to the present invention is not limited to such order. For example, the diagnostic flow for heart disease may be followed by or simultaneous with the diagnostic flow for AD. In the case that one of the diagnostic flow for AD and the diagnostic flow for heart disease is performed prior to the other, the feature described in Embodiments 1 and 2 with respect to the order of the steps (S11 to S15, S21 to S25) is also applied to the present embodiment.

In the case that the diagnostic flow for AD and the diagnostic flow for heart disease are simultaneously performed, it is only needed for the steps that the step of acquiring a threshold (S11, S21) and the step of measuring (S12, S22) are performed before the step of determining (S13, S23) is performed. Accordingly, the step of acquiring a threshold (S11, S21) may be performed simultaneously with the step of measuring (S12, S22), or after the step of measuring (S12, S22). For example, the diagnosis system according to the present invention may be such that thresholds for all urine samples to be determined are acquired at once in accordance with identification information input by a user (S11, S21), the amount of a urine biomarker is measured for all urine samples (S12, S22), determination is made for each of the urine samples on whether the subject suffers from AD/heart disease (S13, S23), and all the determination results may be indicated (S14, S24).

The step of indicating a determination result (S14, S24) and the step of determining (S15, S25) may be, for example, such that determination is made for all urine samples designated (S15, S25) in accordance with identification information input by a user, and after the completion the determination results are indicated (S14, S24). Other features described in Embodiments 1 and 2 with respect to thresholds are also applied to the present embodiment.

Hereinafter, Examples are described as embodiments of the present invention; however, they do not limit the scope of the invention described in the appended claims in any manner.

### Examples

### <Preparation of urine samples>

### (1) Enrichment treatment (concentration and partial purification)

Protein in urine was concentrated basically in accordance with a method described in Kidney International (2010) 77: 736-742.

From a test subject, 25 ml of urine was collected, and centrifuged (17,000 g, 25°C, 10 minutes) to collect the supernatant (SN1). To the precipitate, 250 µL of buffer (10 mM Tris solution (pH 7.6), 200 mg/ml dithiothreitol, 250 mM sucrose) was added to suspend the precipitate, and the resultant was left to stand at 37°C for 10 minutes. The suspension was centrifuged (17,000 g, 25°C, 10 minutes) to collect the supernatant (SN2). The supernatant (SN1) and the supernatant (SN2) were mixed together, to which 25 ml of Total Exosome Isolation reagent (Thermo Fisher Scientific K.K.) was added to mix together, and the resultant was left to stand at room temperature for 1 hour. The mixed solution was centrifuged (10,000 g, 4°C, 60 minutes), and the precipitate was resuspended in 100 µL of D-PBS(-) to afford a partially purified fraction of urine protein. The partially purified fraction obtained was centrifuged (10,000 g, 4°C, 15 minutes) to collect the supernatant (SN3). The supernatant (SN3) was diluted by 2-fold with D-PBS(-), and the resultant was loaded on a gel filtration column (Sephacryl S-300). A fraction eluted at void time was collected and centrifuged (10,000 g, 4°C, 15 minutes). The supernatant (SN4) was concentrated to 50 µL with an ultrafiltration filter (30K MWCO).

### (2) Extraction treatment

The urine protein obtained through the enrichment treatment described above may be present in a urine protein-containing complex, including extracellular vesicles. To extract the urine protein from a urine protein-containing complex (including extracellular vesicles), an alkali extraction process or a freeze-thaw extraction process was used.

Alkali extraction process: to 50 µL of a sample solution derived from urine, 5 µL of 5% Triton X-305 and 5 µL of 4 N NaOH were added, and the resultant was left to stand on ice for 20 minutes; thereafter, 5 µL of 4 N HCl/1 M HEPES solution was added thereto to neutralize.

Freeze-thaw extraction process: 50 µL of a sample solution derived from urine was frozen at -25°C and stored for 2 weeks; to the frozen sample, 5 µL of 5% Triton X-305 was added, and 5 µL of 10% Triton X-100 was further added thereto, and the resultant was left to stand to thaw on ice for 20 minutes.

### <Sandwich ELISA>

### (3) Immobilization of antibody

An antibody for urine protein (hereinafter, referred to as "capture antibody") was immobilized on a 96-well microtiter plate.

D-PBS(-) was used to set the concentration of the capture antibody to 50 µg/ml. Into the wells, 100 µL of the capture antibody solution was aliquoted, and incubated at 4°C overnight to immobilize. The resultant was washed once with washing solution (D-PBS(-) containing 0.05% Tween20), and 300 µL of blocking solution (D-PBS(-) containing 1% bovine serum albumin (BSA)) was then added thereto for blocking. The blocking solution was removed, and the 96-well microtiter plate was then dried in an incubator at 25°C to afford an antibody-immobilized plate. The antibody-immobilized plate was stored at 4°C until use.

### (4) Labeling of detection antibody

An antibody which binds to an epitope differing from an epitope to which the capture antibody binds on urine protein (hereinafter, referred to as "detection antibody") was labeled with horseradish peroxidase (HRP) via a biotin-streptavidin complex.

Basically in accordance with an instruction attached to a Biotin-Labeling Kit-NH2 (DOJINDO LABORATORIES), 50 µg of the detection antibody was labeled with biotin to afford a biotin-labeled antibody. The biotin-labeled antibody was reacted with HRP-labeled streptavidin during ELISA measurement to form an HRP-labeled detection antibody.

### (5) ELISA measurement

A urine sample containing urine protein and a purified product of the urine protein (standard sample) were added to the antibody-immobilized plate, and the antibody-immobilized plate was shaken at 25°C for 1 hour for reaction (primary reaction). The sample solution was removed (B/F separation), and the plate was washed with washing solution three times. To the plate, 100 µL of diluted solution of the biotin-labeled antibody, which had been prepared by diluting the biotin-labeled antibody with ELISA buffer (D-PBS(-) containing 1% BSA, 0.05% Tween20, and 0.05% ProClin 300) to concentrations of 0.1 to 0.25 µg/ml, was added, and the plate was shaken at 25°C for 1 hour for reaction (secondary reaction). The diluted solution was removed, and the plate was washed with washing solution three times. Thereto, 100 µL of HRP-labeled streptavidin solution diluted in advance with ELISA buffer was added, and the plate was shaken at 25°C for 1 hour for reaction (tertiary reaction). The HRP-labeled streptavidin solution was removed, and the plate was washed with washing solution three times. To the plate, 3,3',5,5'-tetramethylbenzidine (TMB) solution was added and allowed to undergo coloring reaction at room temperature for 15 minutes, and 100 µL of 1 N sulfuric acid was added thereto to quench the reaction. The absorbance at 450 nm (reference wavelength: 650 nm) was measured for each well of the plate by using a microplate reader.

### [Example 1]

### <Search for AD-associated urine protein>

Urines were collected from three AD patients. In addition, urines were collected from four healthy subjects in thirties to sixties. In accordance with (1) Enrichment treatment described above, 25 ml of each urine from the AD group (n = 3) and the healthy subject group (n = 4) was concentrated and partially purified, and urine protein was extracted in accordance with (2) Extraction treatment described above to prepare urine samples. The protein concentration of each urine sample prepared was quantified by using a BCA method with a Micro BCA (TM) Protein Assay Kit (Thermo Fisher Scientific K.K.).

Proteomics analysis was performed by Medical ProteoScope Co., Ltd. (Advanced Medical Research Center, Yokohama City University, 3-9, Fukuura, Kanazawa-ku, Yokohama city, Kanagawa prefecture, Japan). To a urine sample containing approximately 0.4 to 1 µg of protein, trichloroacetic acid was added (final concentration: 10%) to precipitate the protein. Dissolving solution (containing 8 M urea) was added to the precipitated protein to dissolve it, and trypsin was then added thereto to decompose the urine protein into peptide. The resulting peptide solution was subjected to LC/MS/MS with the mass spectrometer LTQ-Orbitrap Velos and the liquid chromatograph Ultimate 3000. The LC/MS/MS data acquired were analyzed by using the software Mascot ver. 2.4, and as a result 1200 urine proteins were identified. Comparison of peptide analysis data for the AD group with peptide analysis data for the non-AD group using the software Scaffold 3.0 specified 23 AD-specific urine proteins as candidate substances for biomarkers for AD diagnosis.

### <Identification of AD-associated urine protein (1)>

### (Collection of urine)

Urines were collected from AD patients (n = 5). These AD patients are elderly, and most of them (n = 3) were affected by complication (ischaemic heart disease and hypertension). For use as standard samples, urines were collected not only from healthy subjects (n = 12) in various ages, but also from cardiac hypertrophy patients (n = 3) and unstable angina patients (n = 3) who were elderly and affected by hypertension and further affected by heart disease similar to ischaemic heart disease (CHD). Table 4 shows providers of the urine samples.

### [Table 4]

| Provider | No. | Age/sex | Complication |
|---|---|---|---|
| AD patient | 1 | 87/M | ischaemic heart disease, hypertension |
| | 2 | 79/F | none |
| | 3 | 77/F | ischaemic heart disease, hypertension |
| | 4 | 76/F | ischaemic heart disease, hypertension |
| | 5 | 67/M | chronic gastritis |
| Healthy subject (HS) | 1 | 30/M | |
| | 2 | 30/M | |
| | 3 | 36/M | |
| | 4 | 49/M | |
| | 5 | 49/M | |
| | 6 | 44/M | |
| | 7 | 54/M | |
| | 8 | 50/M | |
| | 9 | 52/M | |
| | 10 | 68/M | |
| | 11 | 60/M | |
| | 12 | 61/M | |
| Cardiac hypertrophy (HT) patient | 1 | 80/F | ischaemic heart disease, hypertension |
| | 2 | 80/F | ischaemic heart disease, hypertension |
| | 3 | 70/F | ischaemic heart disease, hypertension |
| Unstable angina (Ang) patient | 1 | 79/F | ischaemic heart disease, hypertension |
| | 2 | 82/F | ischaemic heart disease, hypertension |
| | 3 | 70/M | ischaemic heart disease, hypertension |

| | | | |
|---|---|---|---|
| AD patients (n = 5, mean age: 77.2); CHD patients (HT patients and Ang patients) (n = 6, mean age: 76.8); HS (n = 12, mean age: 48.6). MMSE scores for the AD patients were 14 to 18. | | | |

### (Preparation of urine samples)

Basically in accordance with (1) Enrichment treatment described above, 500 µL of each urine collected from the AD group (n = 5), the healthy subject group (n = 12), and the CHD group (HT patients and Ang patients) (n = 6) was concentrated and partially purified by 10-fold, and urine protein was extracted therefrom in accordance with (2) Extraction treatment described above (alkali extraction process) to prepare urine samples. By mixing 50 µL of a sample prepared and 50 µL of ELISA buffer (D-PBS(-) containing 1% BSA, 0.05% Tween20, and 0.05% ProClin 300) together, urine samples for ELISA were prepared.

### (ELISA measurement)

Among the 23 urine proteins specified in the proteomics analysis, 22 urine proteins other than ApoD were used as factors, and the concentration of each factor in each urine sample was measured in accordance with the method described in the section "Sandwich ELISA". Here, for urine proteins for which an ELISA measurement kit was commercially available among the 22 urine proteins, measurement was performed in accordance with an instruction attached to the kit.

### (Creatinine correction)

To correct the variation of urine protein concentrations in spot urine, urinary creatinine correction was performed. Amounts of urinary creatinine were measured by using a Urinary Creatinine detection kit (Cell Biolabs, Inc.). Measurement results (concentrations) for urine protein were corrected based on the total amount of creatinine per day (1 g).

### (Analysis)

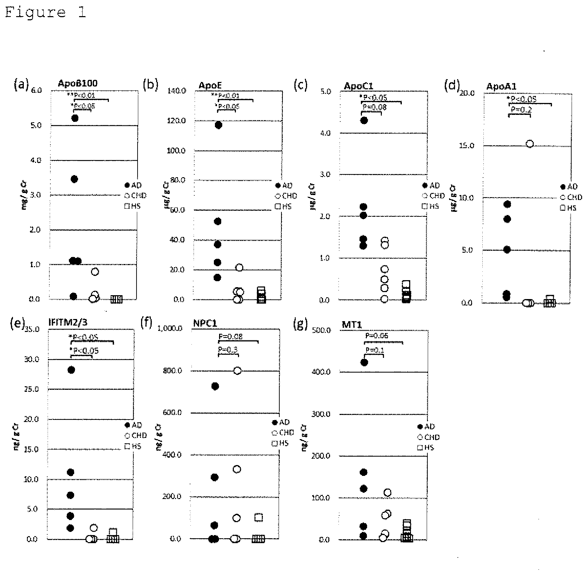

By using the Welch's t-test, statistically significant difference was examined between the AD group and the healthy subject group or the CHD group. As a result, a significant difference or significant tendency was found for seven urine proteins between the results for the AD group and the results for the healthy subject group (Figure 1).

ApoA-I, ApoB100, ApoC-I, and ApoE are each an apolipoprotein known to be a cholesterol transporter in blood. In comparing concentrations of ApoA-I, ApoB100, ApoC-I, and ApoE in urine samples between the AD group and the HS group, the AD group exhibited significantly higher values for all cases (Figure 1 (a) to (d)). In comparing concentrations of ApoB100 and ApoE in urine samples between the AD group and the CHD group, the AD group exhibited significantly higher values for both cases (Figure 1 (a) and (b)).

ApoA-I, ApoB100, ApoC-I, and ApoE are each an apolipoprotein produced in the liver or small intestine and contained, for example, in HDL, chylomicron, VLDL, or LDL. These apolipoproteins have family proteins having a common feature regarding the synthesizing organ and localization (Table 1).

Regarding ApoB-100, ApoE, ApoC-I, and ApoA-I, there are reports that their blood concentrations are changed in an AD group (Non-Patent Literatures 3 to 6). Non-Patent Literatures 3 to 6 show comparison between an AD group and a non-AD group, and demonstrate that blood concentrations of ApoB-100, ApoE, ApoC-I, and ApoA-I were significantly higher in the AD group; however, the difference is insufficient for clinical use as a biomarker. In contrast, Example 1, in which ApoB-100, ApoE, ApoC-I, or ApoA-I in urine samples was used as a biomarker, demonstrates that they can be measured with a large concentration difference (Figure 1 (a) to (d)).

IFITM2/3 is an intracellular cholesterol transport-related protein. In comparing the AD group with the HS group, and comparing the AD group with the CHD group, the AD group exhibited significantly higher values for IFITM2/3 in each comparison (Figure 1 (e)). NPC1 is also known to be an intracellular cholesterol transport-related protein. In comparing the AD group with the HS group for NPC1, the AD group exhibited higher values (Figure 1 (f)).

Although it is known that increased RNA expressions of IFITM2/3 and NPC1 are found in AD postmortem brains (Table 2), this analysis method requires biopsying of brain samples, and thus is not applicable to diagnosis for living subjects. These proteins are usually localized in the endoplasmic reticulum in cells, and hence it has been believed to be less possible that they are released from cells in the brain and pass through the blood-brain barrier. In fact, there has been no report with focus on intracellular cholesterol transport-related protein in the extracellular body fluid to monitor the state in the brain, as far as the present inventors know. Unexpectedly, Example 1 has revealed that the contents of IFITM2/3 and NPC1 significantly changed in relation to AD in the extracellular body fluid, furthermore, in urine, and demonstrated that intracellular cholesterol transport-related proteins in urine are useful for AD diagnosis.

MT is known to be a protein to chelate heavy metal such as zinc, copper, and cadmium. In comparing the AD group with the HS group for MT, the AD group exhibited higher values (Figure 1 (g)).

It has been reported that increased expression of MT1 was found in AD postmortem brains (J. Chem. Neuroanat. (1998) 15: 21-26). However, this analysis method requires biopsying of brain samples, and thus is not applicable to diagnosis for living subjects. In addition, it has been reported that in comparing an AD group with a non-AD group for MT concentrations in blood, almost no difference was found between the groups (Brain Research (2010) 1319: 118-1130). Unexpectedly, Example 1 has revealed that the amount of MT in urine significantly changed in relation to AD, and demonstrated that MT in urine is useful for AD diagnosis.

Table 5 shows results of comparison of the AD group with the HS group for various urine proteins. Table 5 also shows antibodies or kits and standard samples used in ELISA measurement for the urine proteins.

**[Table 5]**

| Urine protein | Antibody or kit | Standard sample | Welch's t-test |
|---|---|---|---|
| ApoA-1 | Quantikine® ELISA Human Apolipoprotein A-I/ApoA1 Immunoassay | content of kit | with significant difference (p<0.05) |
| ApoB-100 | Anti-ApoB-100 mouse monoclonal antibody (JIH)(prepared in-house) | Human Apolipoprotein B (APOB 100) - Purified | with significant difference (P<0.01) |
| | Anti-ApoCl rabbit polyclonal antibody (BM2150) | | |
| ApoC-I | Anti-ApoC1 rabbit polyclonal antibody (ab207931) | Human ApoC1 recombinant protein (ATGP2554) | with significant difference (P<0.05) |
| ApoE | ApoE4/Pan-ApoE ELISA Kit (No. 7635) | content of kit | with significant difference (P<0.01) |
| IFITM2/3 | Anti-Human IFITM2/IFITM3 Antibody, Polyclonal Goat IgG (AF4834) | IFITM2 (Human) Recombinant Protein with GST-tag at N-terminal (H00010581-P02) | with significant difference (P<0.01) |
| NPC1 | Anti-human NPC1 mouse monoclonal antibody (8D10G3) | Human NPC1 recombinant protein (23-269) | with significant tendency (P<0.08) |
| | Anti-human NPC1 mouse monoclonal antibody (4H2) | (prepared in-house) | |
| MT | Human Metallothionein Sandwich ELISA Kit (LS-F10296) | content of kit | with significant tendency (P<0.06) |

| | | | |
|---|---|---|---|
| The anti-ApoB-100 mouse monoclonal antibody (JIH) was prepared by using a known immunological approach. Human NPC1 recombinant protein (23-269) was prepared by using a known recombinant technique. | | | |

### [Example 2]

### <Method for assisting diagnosis using combination of urine biomarkers>

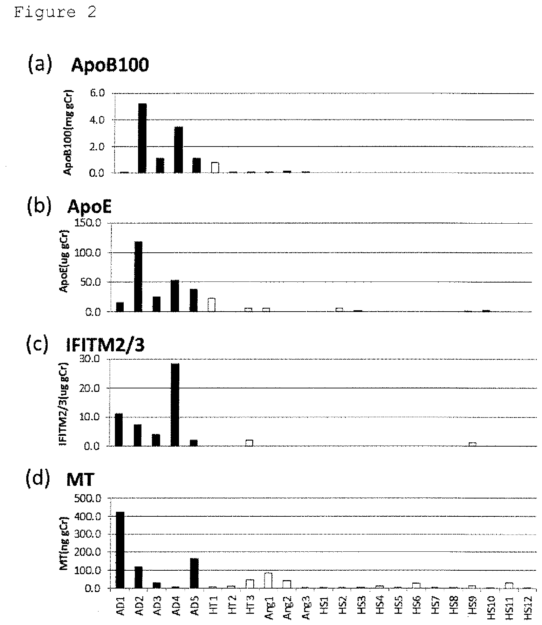

In Example 2, concentrations of IFITM2/3, ApoB-100, ApoE, and MT in urine samples were measured (Figure 2) basically in the same manner as in Example 1.

### (2-1) Combination of IFITM2/3 and ApoB-100:

In first diagnosis, the urine biomarker IFITM2/3 was used, and the first threshold was set to 3.5 [µg/gCr]. In second diagnosis, the urine biomarker ApoB-100 was used, and the second threshold was set to 1.0 [mg/gCr].

The determination criteria were as follows:
(determination 1) suffering from AD (positive) if the measured value was higher than the first threshold;
(determination 2) suffering from AD (positive) if the measured value was higher than the second threshold; and
(final determination) suffering from AD (positive) if either one of results of determination 1 and determination 2 was positive.

In the first method for assisting diagnosis using the urine biomarker IFITM2/3, measured values for the HS group and the CHD group were all equal to or lower than the first threshold (Figure 2 (c)). In the AD group, the AD patients 1 to 4 each exhibited a measured value higher than the first threshold, and determined to be positive (Figure 2 (c), Table 6). In the second method for assisting diagnosis using the urine biomarker ApoB-100, measured values for the HS group and the CHD group were all equal to or lower than the second threshold (Figure 2 (a)). In the AD group, the AD patients 2 to 5 each exhibited a measured value higher than the second threshold, and determined to be positive (Figure 2 (a), Table 6).

According to the above determination criteria, all of the subjects in the AD group were finally determined to be positive (Table 6).

**[Table 6]**

| | AD patient 1 | AD patient 2 | AD patient 3 | AD patient 4 | AD patient 5 |
|---|---|---|---|---|---|
| Determination 1 | positive | positive | positive | positive | - |
| Determination 2 | - | positive | positive | positive | positive |
| Final determination | positive | positive | positive | positive | positive |

### (2-2) Combination of IFITM2/3 and ApoE:

In first diagnosis, the urine biomarker IFITM2/3 was used, and the first threshold was set to 3.5 [µg/gCr]. In second diagnosis, the urine biomarker ApoE was used, and the second threshold was set to 25 [µg/gCr]. The same determination criteria as in Example 2-1 were used.

See Example 2-1 for results of determination 1 in the first method for assisting diagnosis. In the second method for assisting diagnosis using the urine biomarker ApoE, measured values for the HS group and the CHD group were all equal to or lower than the second threshold (Figure 2 (b)). In the AD group, the AD patients 2 to 5 each exhibited a measured value higher than the second threshold, and determined to be positive (Figure 2 (b), Table 7).

According to the determination criteria, all of the subjects in the AD group were finally determined to be positive (Table 7).

**[Table 7]**

| | AD patient 1 | AD patient 2 | AD patient 3 | AD patient 4 | AD patient 5 |
|---|---|---|---|---|---|
| Determination 1 | positive | positive | positive | positive | - |
| Determination 2 | - | positive | positive | positive | positive |
| Final determination | positive | positive | positive | positive | positive |

### (2-3) Combination of IFITM2/3 and MT:

In first diagnosis, the urine biomarker IFITM2/3 was used, and the first threshold was set to 3.5 [µg/gCr]. In second diagnosis, the urine biomarker MT was used, and the second threshold was set to 80 [ng/gCr]. The same determination criteria as in Example 2-1 were used.

See Example 2-1 for results of determination 1 in the first method for assisting diagnosis. In the second method for assisting diagnosis using the urine biomarker MT, measured values for the HS group and the CHD group were all equal to or lower than the second threshold (Figure 2 (d)). In the AD group, the AD patients 1, 2, and 5 each exhibited a measured value higher than the second threshold, and determined to be positive (Figure 2 (d), Table 8).

According to the above determination criteria, all of the subjects in the AD group were finally determined to be positive (Table 8).

**[Table 8]**

| | AD patient 1 | AD patient 2 | AD patient 3 | AD patient 4 | AD patient 5 |
|---|---|---|---|---|---|
| Determination 1 | positive | positive | positive | positive | - |
| Determination 2 | positive | positive | - | - | positive |
| Final determination | positive | positive | positive | positive | positive |

### (2-4) Combination of MT and ApoB-100:

In first diagnosis, the urine biomarker MT was used, and the first threshold was set to 80 [ng/gCr]. In second diagnosis, the urine biomarker ApoB-100 was used, and the second threshold was set to 1.0 [mg/gCr]. The same determination criteria as in Example 2-1 were used.

In the first method for assisting diagnosis using the urine biomarker MT, measured values for the HS group and the CHD group were all equal to or lower than the first threshold (Figure 2 (d)). In the AD group, the AD patients 1, 2, and 5 each exhibited a measured value higher than the first threshold, and determined to be positive (Figure 2 (d), Table 9). In the second method for assisting diagnosis using the urine biomarker ApoB-100, measured values for the HS group and the CHD group were all equal to or lower than the second threshold (Figure 2 (a)). In the AD group, the AD patients 2 to 5 each exhibited a measured value higher than the second threshold, and determined to be positive (Figure 2 (a), Table 9).

According to the determination criteria, all of the subjects in the AD group were finally determined to be positive (Table 9).

**[Table 9]**

| | AD patient 1 | AD patient 2 | AD patient 3 | AD patient 4 | AD patient 5 |
|---|---|---|---|---|---|
| Determination 1 | positive | positive | - | - | positive |
| Determination 2 | - | positive | positive | positive | positive |
| Final determination | positive | positive | positive | positive | positive |

### (2-5) Combination of MT and ApoE:

In first diagnosis, the urine biomarker MT was used, and the first threshold was set to 80 [ng/gCr]. In second diagnosis, the urine biomarker ApoE was used, and the second threshold was set to 25 [µg/gCr]. The same determination criteria as in Example 2-1 were used.

See Example 2-4 for results of determination 1 in the first method for assisting diagnosis. In the second method for assisting diagnosis using the urine biomarker ApoE, measured values for the HS group and the CHD group were all equal to or lower than the second threshold (Figure 2 (b)). In the AD group, the AD patients 2 to 5 each exhibited a measured value higher than the second threshold, and determined to be positive (Figure 2 (b), Table 10).

According to the determination criteria, all of the subjects in the AD group were finally determined to be positive (Table 10).

**[Table 10]**

| | AD patient 1 | AD patient 2 | AD patient 3 | AD patient 4 | AD patient 5 |
|---|---|---|---|---|---|
| Determination 1 | positive | positive | - | - | positive |
| Determination 2 | - | positive | positive | positive | positive |
| Final determination | positive | positive | positive | positive | positive |

It was demonstrated that the method for assisting diagnosis using two urine biomarkers enables AD diagnosis with better sensitivity than the method for assisting diagnosis using one urine biomarker. In Example 2, the sensitivity of the method for assisting diagnosis using two urine biomarkers was 100%.

### [Example 3]

### <Influence of variation of amount of urine protein>

To correct the variation of urine protein concentrations in spot urine, urinary creatinine correction was performed in Examples 1 and 2. Influence of the variation of the amount of urine protein in spot urine on urine biomarker measurement was examined.

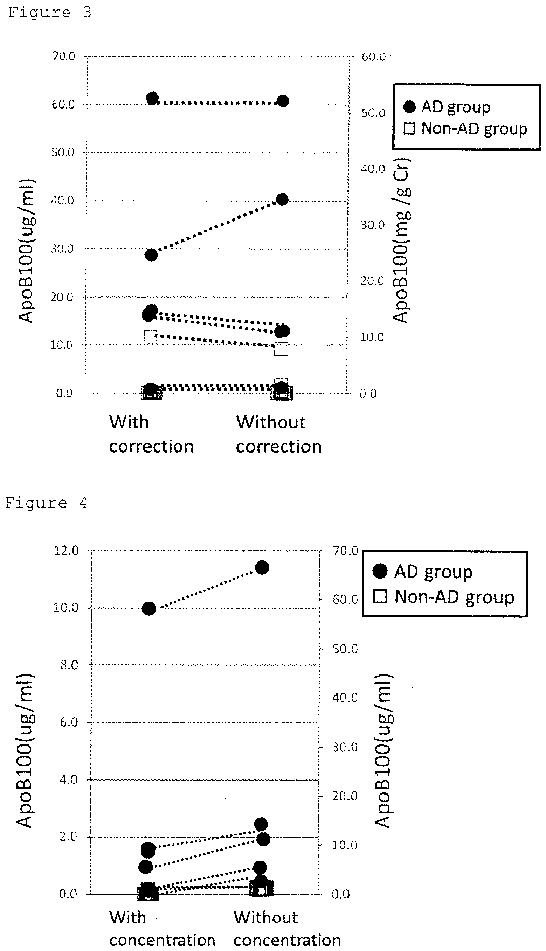

Preparation of urine samples and ELISA measurement were performed with spot urine collected from the AD group and the non-AD group basically in the same manner as in Example 1, and concentrations of ApoB-100 (without correction) were measured. Further, urinary creatinine correction was performed by using the same manner as in Example 1, and ApoB-100 concentrations (with correction) were calculated. The results are shown in Figure 3. As shown in Figure 3, measurement results for the AD group and the non-AD group were found to be hardly influenced by the presence or absence of creatinine correction.

The urine biomarkers specified in Example 1 other than ApoB-100 were similarly examined with respect to the presence or absence of creatinine correction, and almost no influence was found in measurement results for all of the urine biomarkers (data not shown). Accordingly, the method for assisting diagnosis according to the present invention using a urine biomarker does not require urinary creatinine correction even in using spot urine as a urine sample, and thus can be implemented in a simple manner.

### [Example 4]

### <Preparation of urine samples: influence of enrichment treatment>

Each urine (500 µL) collected from the AD group and the non-AD group was concentrated by 10-fold basically in accordance with (1) Enrichment treatment described above to prepare urine samples (with concentration), and urine (50 µL) collected from each subject was directly used to prepare urine samples (without concentration), and ApoB-100 concentrations were measured for the urine samples (with concentration) and the urine samples (without concentration) basically in the same manner as in Example 1 except that creatinine correction was not performed (Figure 4). Regardless of the presence or absence of enrichment treatment, the AD group exhibited a specific tendency of increased ApoB-100 concentrations compared with those for the non-AD group. Thus, the urine protein ApoB-100 was demonstrated to be measurable regardless of the presence or absence of enrichment treatment.

### [Example 5]

### <Preparation of urine samples: influence of extraction treatment>

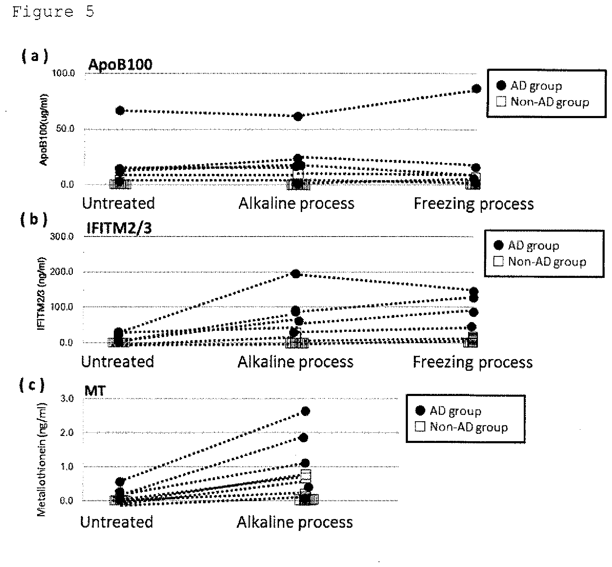

Each urine collected for the AD group and the non-AD group was concentrated basically in accordance with (1) Enrichment treatment described above. The concentrated samples were directly used as urine samples (untreated) without (2) Extraction treatment described above, or subjected to extraction treatment with the alkali extraction process to prepare urine samples (alkaline process), or subjected to extraction treatment with the freeze-thaw process to prepare urine samples (freezing process). Concentrations of ApoB-100, IFITM2/3, and MT were measured for the urine samples prepared basically by using the same manner as in Example 1 except that creatinine correction was not performed (Figure 5).

When the apolipoprotein ApoB-100 in urine was measured (Figure 5 (a)), almost no difference in measurement results was found between the urine samples without extraction treatment (untreated) and the urine samples subjected to the alkali extraction process (alkaline process). Similarly, almost no difference in measurement results was found between the urine samples (untreated) and the samples subjected to the freeze-thaw process (freezing process). Accordingly, extraction treatment for urine samples may be performed or not in the method for assisting diagnosis using the apolipoprotein ApoB-100 in urine as a urine biomarker.

When the cholesterol transport-related factor IFITM2/3 in urine was measured (Figure 5 (b)), large difference in measurement results was found between the urine samples without extraction treatment (untreated) and the urine samples subjected to extraction treatment (alkaline process/freezing process). Almost no signal (absorbance) was obtained for the urine samples (untreated) in ELISA measurement. In contrast, signals were obtained for the urine samples subjected to extraction treatment. For the signals obtained, almost no difference was found between the alkali extraction process and the freeze/thaw process. Accordingly, urine samples subjected to extraction treatment are preferred in the case that the cholesterol transport-related factor IFITM2/3 in urine is a target for measurement and IFITM2/3 is measured by using combination of an Anti-Human IFITM2/IFITM3 Antibody and a polyclonal Goat IgG (AF4834).

When MT in urine was measured (Figure 5 (c)), large difference in measurement results was found between the urine samples without extraction treatment (untreated) and the urine samples subjected to extraction treatment (freezing process). Almost no signal (absorbance) was obtained for the urine samples (untreated) in ELISA measurement. In contrast, signals were obtained for the urine samples subjected to extraction treatment. Accordingly, urine samples subjected to extraction treatment are preferred in the case that MT in urine is a target for measurement and MT is measured by using a Human Metallothionein Sandwich ELISA Kit (LS-F10296).

### [Example 6]

### <Method for assisting diagnosis utilizing co-localization of urine biomarkers>

Each urine collected from the AD group, the HT patient group, the Ang patient group, and the HS group was concentrated and partially purified basically in accordance with (1) Enrichment treatment described above to prepare samples, which were used as urine samples. Here, (2) Extraction treatment described above was not performed. An anti-ApoE antibody (Anti-Human ApoE Antibody, monoclonal mouse IgG (WUE-4 NB110 60531)) and an anti-ApoC-I antibody (anti-ApoC1 rabbit polyclonal antibody (ab207931)) were each immobilized on a 96-well microtiter plate in accordance with (3) Immobilization of antibody described above. An anti-ApoB-100 mouse monoclonal antibody (JIH) was labeled with biotin in accordance with (4) Labeling of detection antibody described above.

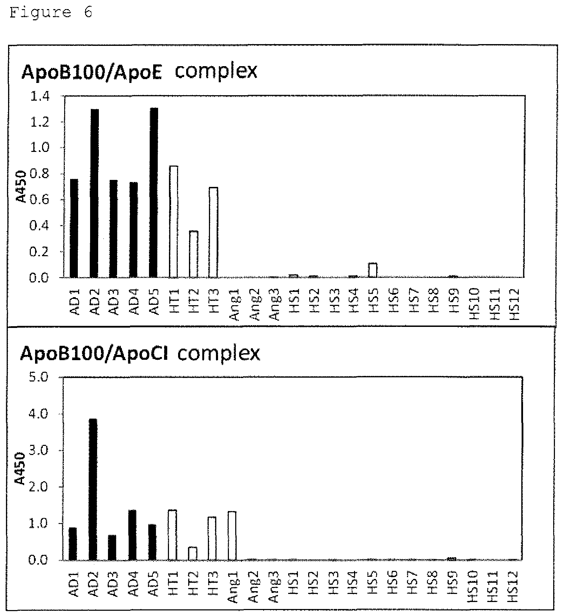

By using the urine samples prepared, the 96-well microtiter plate including the anti-ApoE antibody immobilized thereon, and the biotin-labeled anti-ApoB-100 antibody, a complex including ApoB-100 and ApoE (hereinafter, also referred to as "ApoB-100/ApoE complex") was measured with sandwich ELISA in accordance with (5) ELISA measurement described above. The measurement results (absorbance: A450) are shown in Figure 6. An absorbance signal detected in this sandwich ELISA suggests the presence of a urine protein-containing complex including ApoB-100 and ApoE co-localized therein in a urine sample prepared. In comparing the AD group with the HS group for the ApoB-100/ApoE complex, the AD group exhibited significantly higher values (P = 0.001). This measurement based on co-localization of combination of urine biomarkers can enable AD diagnosis with higher sensitivity than in AD diagnosis based on measurement of one urine biomarker (e.g., Figure 1), and AD diagnosis based on sequential measurement of two urine biomarkers (e.g., Figure 2).

As shown in the top of Figure 6, the ApoB-100/ApoE complex was found in measurement for the HT group among the CHD group (the HT group and the Ang group). On the other hand, the ApoB-100/ApoE complex was not found in measurement for the Ang group. The ApoB-100/ApoE complex is also expected to be a biomarker specific to the HT group. Accordingly, this measurement based on co-localization of combination of urine biomarkers can be useful for diagnosis of AD and HT.

By using the urine samples prepared, the 96-well microtiter plate including the anti-ApoC-I antibody immobilized thereon, and the biotin-labeled anti-ApoB-100 antibody, a complex including ApoB-100 and ApoC-I (hereinafter, also referred to as "ApoB-100/ApoCI complex") was measured with sandwich ELISA in accordance with (5) ELISA measurement described above. The measurement results (absorbance: A450) are shown in Figure 6. An absorbance signal detected in this sandwich ELISA suggests the presence of a urine protein-containing complex including ApoB-100 and ApoC-I co-localized therein in a urine sample prepared. In comparing the AD group with the HS group for the ApoB-100/ApoCI complex, the AD group exhibited significantly higher values (P = 0.029). This measurement based on co-localization of combination of urine biomarkers can enable AD diagnosis with higher sensitivity than in AD diagnosis based on measurement of one urine biomarker or sequential measurement of two urine biomarkers.

As shown in the bottom of Figure 6, the ApoB-100/ApoCI complex was also found in measurement for the CHD group. The ApoB-100/ApoCI complex is also expected to be a biomarker specific to the CHD group. Accordingly, this measurement based on co-localization of combination of urine biomarkers can be useful for diagnosis of AD and CHD.

The method for assisting diagnosis based on co-localization of at least two urine biomarkers, in particular, at least two apolipoproteins, enables diagnosis with higher sensitivity than in methods for assisting diagnosis based on one urine biomarker and methods for assisting diagnosis using at least two urine biomarkers separately.

The method for assisting diagnosis based on co-localization of at least two urine biomarkers, in particular, at least two apolipoproteins, enables diagnosis of heart disease (CHD) including hypertrophy (HT) and unstable angina (Ang).

Thus, another aspect of the present invention provides a method for assisting diagnosis of AD and/or heart disease based on co-localization of at least two urine biomarkers in a urine protein-containing complex. An embodiment of the present invention can provide a method for assisting diagnosis specific to AD through combination with a cognitive function test according to diagnosis criteria of MMSE or the like and brain imaging examination. An embodiment of the present invention can provide a method for assisting diagnosis specific to heart disease through combination with a method for assisting diagnosis such as cardiac imaging examination.

## Claims

1. A method for assisting diagnosis of Alzheimer's disease (AD), the method comprising the steps of:
measuring an amount of a urine biomarker in a urine sample derived from urine collected from a subject; and
determining whether the subject suffers from AD or has a high risk of developing AD based on the amount of the urine biomarker measured, wherein
the urine biomarker is at least one urine protein selected from the group consisting of Apolipoprotein (hereinafter, abbreviated as "Apo") A-I, ApoA-II, ApoA-IV, ApoB-100, ApoB-48, ApoC-I, ApoC-II, ApoC-III, ApoD, ApoE, Interferon-induced transmembrane protein (hereinafter, abbreviated as "IFITM") 1, IFITM2, IFITM3, Neimann-Pick C (hereinafter, abbreviated as "NPC") 1, NPC2, NPC1L1, and Metallothionein (hereinafter, abbreviated as "MT").

2. The method according to claim 1, wherein the step of determining includes comparing the amount of the urine biomarker measured with a threshold corresponding to the amount of the urine biomarker, and
the subject is determined to suffer from AD or have a high risk of developing AD if the amount of the urine biomarker measured is higher than the threshold.

3. The method according to claim 1 or 2, wherein the urine biomarker is at least one urine protein selected from the group consisting of ApoA-I, ApoB-100, ApoC-I, ApoD, ApoE, IFITM2, IFITM3, NPC1, and MT.

4. The method according to any one of claims 1 to 3, wherein pathological condition of AD is dementia caused by AD or mild cognitive impairment caused by AD.

5. The method according to any one of claims 1 to 4, further comprising the step of preparing the urine sample by using urine collected from the subject, wherein
the step of preparing the urine sample includes the step of enriching a urine protein-containing complex derived from the urine.

6. The method according to any one of claims 1 to 5, further comprising the step of preparing the urine sample by using urine collected from a subject, wherein
the step of preparing the urine sample includes the step of extracting the urine biomarker from a urine protein-containing complex derived from the urine.

7. The method according to any one of claims 1 to 5, wherein the step of measuring includes the step of measuring the urine biomarker in a free form.

8. The method according to any one of claims 1 to 7, wherein the urine biomarker is at least two urine proteins selected from the group recited in claim 1.

9. The method according to any one of claims 1 to 5, wherein
the urine biomarker is at least two urine proteins selected from the group recited in claim 1,
the urine sample contains a urine protein-containing complex derived from the urine, and
the at least two urine biomarkers are co-localized in the complex.

10. The method according to claim 9, wherein
the step of measuring is the step of measuring an amount of the urine protein-containing complex, and
the step of determining further includes determining whether the subject suffers from heart disease or has a high risk of developing heart disease.

11. The method according to any one of claims 1 to 10, wherein the step of measuring includes forming a conjugate of the urine biomarker with a reagent for detecting the urine biomarker and detecting a signal reflecting the amount of the urine biomarker derived from the conjugate.

12. The method according to claim 11, wherein the reagent contains at least one probe selected from the group consisting of antibodies, antibody fragments, single-chain antibodies, and aptamers, each for the urine biomarker.

13. The method according to claim 11 or 12, wherein the reagent further contains at least one labeling substance selected from the group consisting of fluorescent substances, radioactive substances, and enzymes.

14. A detection reagent for use in the method according to any one of claims 1 to 13, comprising at least one probe selected from the group consisting of antibodies, antibody fragments, single-chain antibodies, and aptamers, each for at least one urine biomarker selected from the group consisting of ApoA-I, ApoA-II, ApoA-IV, ApoB-100, ApoB-48, ApoC-I, ApoC-II, ApoC-III, ApoD, ApoE, IFITM1, IFITM2, IFITM3, NPC1, NPC2, NPC1L1, and MT.

15. The detection reagent according to claim 14, further comprising at least one labeling substance selected from the group consisting of fluorescent substances, radioactive substances, and enzymes.

16. A diagnosis kit for use in the method according to any one of claims 1 to 13, comprising the detection reagent according to claim 14 or 15.

17. A diagnosis system comprising:
a determination section configured to determine whether a subject suffers from AD or has a high risk of developing AD by comparing an amount of a urine biomarker in a urine sample derived from urine collected from the subject with a threshold corresponding to the amount of the urine biomarker with respect to AD; and
an indication section configured to indicate a determination result from the determination section, wherein
the urine biomarker is at least one urine protein selected from the group consisting of ApoA-I, ApoA-II, ApoA-IV, ApoB-100, ApoB-48, ApoC-I, ApoC-II, ApoC-III, ApoD, ApoE, IFITM1, IFITM2, IFITM3, NPC1, NPC2, NPC1L1, and MT.

18. The diagnosis system according to claim 17, comprising:
a database storing thresholds corresponding to a plurality of the urine biomarkers selected from the group recited in claim 17, wherein
the determination section refers to information including types of the urine biomarker in the urine sample derived from the urine collected from the subject to acquire the corresponding threshold from the database based on the information, and makes determination based on the threshold acquired.

19. The diagnosis system according to claim 17 or 18, wherein
the amount of the urine biomarker in the urine sample is an amount of a urine protein-containing complex including at least two urine biomarkers co-localized therein in the urine sample, and
the threshold is a threshold corresponding to the amount of the urine protein-containing complex including the at least two urine biomarkers co-localized therein.

20. The diagnosis system according to claim 19, wherein the determination section further determines whether the subject suffers from heart disease or has a high risk of developing heart disease by comparing the amount of the urine protein-containing complex including at least two urine biomarkers co-localized therein in the urine sample with a threshold corresponding to the amount of the urine protein-containing complex including the at least two urine biomarkers co-localized therein with respect to heart disease.
